# FASCICULE DE BREVET EUROPEEN

(11) **EP 1 597 364 B1**
(45) Date de publication et mention de la délivrance du brevet: **24.07.2013**
(21) Numéro de dépôt: 04711626.4
(22) Date de dépôt: 17.02.2004
(51) Int. Cl.: C12N 15/01, C12N 9/10, C12P 13/12

(54) **PROCEDE DE PREPARATION DE MICROORGANISMES EVOLUES PERMETTANT LA CREATION OU LA MODIFICATION DE VOIES METABOLIQUES**
VERFAHREN ZUR HERSTELLUNG VON MIKROORGANISMEN, DIE EINER UNTERSCHIEDLICHEN EVOLUTION UNTERWORFEN SIND UND DIE DAS ENSTEHEN ODER DIE ÄNDERUNG VON METABOLISCHEN WEGEN ERLAUBEN
METHOD FOR THE PRODUCTION OF EVOLVED MICROORGANISMS WHICH PERMIT THE GENERATION OR MODIFICATION OF METABOLIC PATHWAYS

(30) Priorité: 18.02.2003 FR 0301924; 14.05.2003 FR 0305768; 14.05.2003 FR 0305769; 06.11.2003 FR 0313054
(43) Date de publication de la demande: 23.11.2005
(60) Demande divisionnaire: 10183261.6
(73) Titulaire: Metabolic Explorer, 63360 Saint Beauzire (FR)
(72) Inventeur: CHATEAU, Michel, F-63200 Riom (FR); GONZALEZ, Benjamin, F-63200 Riom (FR); MEYNIAL-SALLES, Isabelle, F-31450 Fourquevaux (FR); SOUCAILLE, Philippe, Nöel, Paul, F-31450 Deyme (FR); ZINK, Olivier, F-68100 Mulhouse (FR)
(74) Mandataire: Regimbeau
(86) Numéro de dépôt international: PCT/FR2004/000354
(87) Numéro de publication internationale: WO 2004/076659

(56) Documents cités:
- EP-A- 0 519 113
- WO-A-02/18613
- WO-A-93/17112
- WO-A-02/051231
- WO-A-02/083892
- GB-A- 2 075 055
- US-A- 3 071 518
- FLAVIN M ET AL: "ENZYMATIC SYNTHESIS OF HOMOCYSTEINE OR METHIONINE DIRECTLY FROM O-SUCCINYL-HOMOSERINE" BIOCHIMICA ET BIOPHYSICA ACTA, AMSTERDAM, NL, vol. 132, no. 2, 15 mars 1967 (1967-03-15), pages 400-405, XP008028828 ISSN: 0006-3002 cité dans la demande
- SMITH I K ET AL: "UTILIZATION OF S-METHYLCYSTEINE AND METHYLMERCAPTAN BY METHIONINELESS MUTANTS OF NEUROSPORA AND THE PATHWAY OF THEIR CONVERSION TO METHIONINE. II. ENZYME STUDIES" BIOCHIMICA ET BIOPHYSICA ACTA, AMSTERDAM, NL, vol. 184, no. 1, 17 juin 1969 (1969-06-17), pages 130-138, XP008028840 ISSN: 0006-3002 cité dans la demande
- MAY OLIVER ET AL: "Inverting enantioselectivity by directed evolution of hydantoinase for improved production of L-methionine" NATURE BIOTECHNOLOGY, NATURE PUBLISHING, US, vol. 18, no. 3, mars 2000 (2000-03), pages 317-320, XP002154849 ISSN: 1087-0156
- DUCHANGE N ET AL: "E.coli metB and metL (5' end) genes coding for cystathione gamma-synthase and aspartokinase II-homoserine dehydrogenase II" EMBL, 13 juin 1985 (1985-06-13), XP002274156
- KAWASHIMA T ET AL: "Cystathionine beta lyase / O-succinylhomoserine lyase" EMBL, 1 octobre 2001 (2001-10-01), XP002274157
- ARNOLD F H ET AL: "OPTIMIZING INDUSTRIAL ENZYMES BY DIRECTED EVOLUTION" ADVANCES IN BIOCHEMICAL ENGINEERING, BIOTECHNOLOGY, SPRINGER, BERLIN, DE, vol. 58, 1997, pages 1-14, XP000891392 ISSN: 0724-6145
- WEISSBACH H ET AL: "REGULATION OF METHIONINE SYNTHESIS IN ESCHERICHIA COLI" MOLECULAR MICROBIOLOGY, BLACKWELL SCIENTIFIC, OXFORD, GB, vol. 5, no. 7, 1987, pages 1593-1597, XP001037155 ISSN: 0950-382X cité dans la demande
- DATABASE WPI Section Ch, Week 200043 Derwent Publications Ltd., London, GB; Class B05, AN 2000-485354 XP002265367 & JP 2000 157267 A (AJINOMOTO KK) 13 juin 2000 (2000-06-13) cité dans la demande
- KASE H ET AL: "L-METHIONINE PRODUCTION BY METHIONINE ANALOG-RESISTANT MUTANTS OF CORYNEBACTERIUM GLUTAMICUM" AGRICULTURAL AND BIOLOGICAL CHEMISTRY, JAPAN SOC. FOR BIOSCIENCE, BIOTECHNOLOGY AND AGROCHEM. TOKYO, JP, vol. 39, no. 1, 1975, pages 153-160, XP009012948 ISSN: 0002-1369
- NAKAMORI S ET AL: "Mechanism of L-methionine overproduction by Escherichia coli: The replacement of Ser-54 by Asn in the MetJ protein causes the derepression of L-methionine biosynthetic enzymes" APPLIED MICROBIOLOGY AND BIOTECHNOLOGY, SPRINGER VERLAG, BERLIN, DE, vol. 52, no. 2, août 1999 (1999-08), pages 179-185, XP002242975 ISSN: 0175-7598
- MORTLOCK R P: "Metabolic acquisitions through laboratory selection.", ANNUAL REVIEW OF MICROBIOLOGY 1982 LNKD- PUBMED:6816136, vol. 36, 1982, pages 259-284, ISSN: 0066-4227
- CHIRUMAMILLA R R ET AL: "IMPROVING THE QUALITY OF INDUSTRIALLY IMPORTANT ENZYMES BY DIRECTED EVOLUTION", MOLECULAR AND CELLULAR BIOCHEMISTRY, NORWELL, MA, US, vol. 224, no. 1/02, 1 January 2001 (2001-01-01), pages 159-168, XP009002123, ISSN: 0300-8177, DOI: DOI:10.1023/A:1011904405002
- ALLEN M K AND YANOFSKY C: "A BIOCHEMICAL AND GENETIC STUDY OF REVERSION WITH THE A-GENE A-PROTEIN SYSTEM OF ESCHERICHIA COLI TRYPTOPHAN SYNTHETASE", GENETICS, vol. 48, August 1963 (1963-08), pages 1065-1083,
- BARRY G HALL: "Changes in the substrate specificities of an enzyme during directed evolution of new functions", BIOCHEMISTRY, vol. 20, 1981, pages 4042-4049, XP002265867,

### SEQUENCE LISTING

<110> Metabolic Explorer
<120> Procédé de préparation de microorganismes évolués permettant la création ou la modification de voies métaboliques
<130> D20701/ 345773
<150> FR 0301924
   <151> 2003-02-18
<150> FR 0305768
   <151> 2003-05-14
<150> FR 0305769
   <151> 2003-05-14
<150> FR 0313054
   <151> 2003-11-06
<160> 42
<170> PatentIn version 3.1
<210> 1
   <211> 100
   <212> DNA
   <213> Artificial sequence
<220>
   <223> DmetER
<400> 1
   tacccccgac gcaagttctg cgccgcctgc accatgttcg ccagtgccgc gcgggtttct      60
   ggccagccgc gcgttttcag catatgaata tcctccttag                           100
<210> 2
   <211> 100
   <212> DNA
   <213> Artificial Sequence
<220>
   <223> DmetEF
<400> 2
   tgacaatatt gaatcacacc ctcggtttcc ctcgcgttgg cctgcgtcgc gagctgaaaa      60
   aagcgcaaga aagttattgg tgtaggctgg agctgcttcg                           100
<210> 3
   <211> 30
   <212> DNA
   <213> Artificial Sequence
<220>
   <223> MetER
<400> 3
   ggtttaagca gtatggtggg aagaagtcgc 30
<210> 4
   <211> 30
   <212> DNA
   <213> Artificial Sequence
<220>
   <223> MetEF
<400> 4
   cccggggatg aataaacttg ccgccttccc 30
<210> 5
   <211> 1161
   <212> DNA
   <213> Escherichia coli
<400> 5
   atgacgcgta aacaggccac catcgcagtg cgtagcgggt taaatgacga cgaacagtat 60
   ggttgcgttg tcccaccgat ccatctttcc agcacctata actttaccgg atttaatgaa 120
   ccgcgcgcgc atgattactc gcgtcgcggc aacccaacgc gcgatgtggt tcagcgtgcg 180
   ctggcagaac tggaaggtgg tgctggtgca gtacttacta ataccggcat gtccgcgatt 240
   cacctggtaa cgaccgtctt tttgaaacct ggcgatctgc tggttgcgcc gcacgactgc 300
   tacggcggta gctatcgcct gttcgacagt ctggcgaaac gcggttgcta tcgcgtgttg 360
   tttgttgatc aaggcgatga acaggcatta cgggcagcgc tggcagaaaa acccaaactg 420
   gtactggtag aaagcccaag taatccattg ttacgcgtcg tggatattgc gaaaatctgc 480
   catctggcaa gggaagtcgg ggcggtgagc gtggtggata acaccttctt aagcccggca 540
   ttacaaaatc cgctggcatt aggtgccgat ctggtgttgc attcatgcac gaaatatctg 600
   aacggtcact cagacgtagt ggccggcgtg gtgattgcta aagacccgga cgttgtcact 660
   gaactggcct ggtgggcaaa caatattggc gtgacgggcg gcgcgtttga cagctatctg 720
   ctgctacgtg ggttgcgaac gctggtgccg cgtatggagc tggcgcagcg caacgcgcag 780
   gcgattgtga aatacctgca aacccagccg ttggtgaaaa aactgtatca cccgtcgttg 840
   ccggaaaatc aggggcatga aattgccgcg cgccagcaaa aaggctttgg cgcaatgttg 900
   agttttgaac tggatggcga tgagcagacg ctgcgtcgtt tcctgggcgg gctgtcgttg 960
   tttacgctgg cggaatcatt agggggagtg gaaagtttaa tctctcacgc cgcaaccatg 1020
   acacatgcag gcatggcacc agaagcgcgt gctgccgccg ggatctccga gacgctgctg 1080
   cgtatctcca ccggtattga agatggcgaa gatttaattg ccgacctgga aaatggcttc 1140
   cgggctgcaa acaaggggta a 1161
<210> 6
   <211> 10
   <212> PRT
   <213> Escherichia coli
<400> 6
   Met Glu Thr Thr His Arg Ala Arg Gly Leu
   1 5 10
<210> 7
   <211> 1161
   <212> DNA
   <213> Escherichia coli
<400> 7
   atgacgcgta aacaggccac catcgcagtg cgtagcgggt taaatgacga cgaacagtat 60
   ggttgcgttg tcccaccgat ccatctttcc agcacctata actttaccgg atttaatgaa 120
   ccgcgcgcgc atgattactc gcgtcgcggc aacccaacgc gcgatgtggt tcagcgtgcg 180
   ctggcagaac tggaaggtgg tgctggtgca gtacttacta ataccggcat gtccgcgatt 240
   cacctggtaa cgaccgtctt tttgaaacct ggcgatctgc tggttgcgcc gcacgactgc 300
   tacggcggta gctatcgcct gttcgacagt ctggcgaaac gcggttgcta tcgcgtgttg 360
   tttgttgatc aaggcgatga acaggcatta cgggcagcgc tggcagaaaa acccaaactg 420
   gtactggtag aaagcccaag taatccattg ttacgcgtcg tggatattgc gaaaatctgc 480
   catctggcaa gggaagtcgg ggcggtgagc gtggtggata acaccttctt aagcccggca 540
   ttacaaaatc cgctggcatt aggtgccgat ctggtgttgc attcatgcac gaaatatctg 600
   aacggtcact cagacgtagt ggccggcgtg gtgattgcta aagacccgga cgttgtcact 660
   gaactggcct ggtgggcaaa caatattggc gtgacgggcg gcgcgtttga cagctatctg 720
   ctgctacgtg ggttgcgaac gctggtgccg cgtatggagc tggcgcagcg caacgcgcag 780
   gcgattgtga aatacctgca aacccagccg ttggtgaaaa aactgtatca cccgtcgttg 840
   ccggaaaatc aggggcatga aattgccgcg cgccagcaaa aaggctttgg cgcaatgttg 900
   agttttgaac tggatggcga tgagcagacg ctgcgtcgtt tcctgggcgg gctgtcgttg 960
   tttacgctgg cggcatcatt agggggagtg gaaagtttaa tctctcacgc cgcaaccatg 1020
   acacatgcag gcatggcacc agaagcgcgt gctgccgccg ggatctccga gacgctgctg 1080
   cgtatctcca ccggtattga agatggcgaa gatttaattg ccgacctgga aaatggcttc 1140
   cgggctgcaa acaaggggta a 1161
<210> 8
   <211> 5
   <212> PRT
   <213> Escherichia coli
<400> 8
   Met Glu Thr Thr His
   1               5
<210> 9
   <211> 30
   <212> DNA
   <213> Artificial Sequence
<220>
   <223> MetJR
<400> 9
   ggtacagaaa ccagcaggct gaggatcagc 30
<210> 10
   <211> 100
   <212> DNA
   <213> Artificial Sequence
<220>
   <223> DmetJBF
<400> 10
   tatgcagctg acgacctttc gcccctgcct gcgcaatcac actcattttt accccttgtt 60
   tgcagcccgg aagccatttt caggcaccag agtaaacatt 100
<210> 11
   <211> 30
   <212> DNA
   <213> Artificial Sequence
<220>
   <223> MetCR
<400> 11
   cgtccgggac gccttgatcc cggacgcaac 30
<210> 12
   <211> 32
   <212> DNA
   <213> Artificial Sequence
<220>
   <223> MetCF
<400> 12
   gcgtttacgc agtaaaaaag tcaccagcac gc 32
<210> 13
   <211> 100
   <212> DNA
   <213> Artificial Sequence
<220>
   <223> DmetCR
<400> 13
   ccggcgtcca gatcggcaat cagatcgtcg acatcttcca gaccaatatg caggcgaatc 60
   aaggtcccgc taaaatcgat catatgaata tcctccttag 100
<210> 14
   <211> 100
   <212> DNA
   <213> Artificial Sequence
<220>
   <223> DmetCF
<400> 14
   cggacaaaaa gcttgatact caactggtga atgcaggacg cagcaaaaaa tacactctcg 60
   gcgcggtaaa tagcgtgatt tgtaggctgg agctgcttcg 100
<210> 15
   <211> 100
   <212> DNA
   <213> Artificial Sequence
<220>
   <223> DcysKR
<400> 15
   tgttgcaatt ctttctcagt gaagagatcg gcaaacaatg cggtgcttaa ataacgctca 60
   cccgatgatg gtagaataac catatgaata tcctccttag 100
<210> 16
   <211> 100
   <212> DNA
   <213> Artificial Sequence
<220>
   <223> DcySKF
<400> 16
   agtaagattt ttgaagataa ctcgctgact atcggtcaca cgccgctggt tcgcctgaat 60
   cgcatcggta acggacgcat tgtaggctgg agctgcttcg 100
<210> 17
   <211> 100
   <212> DNA
   <213> Artificial sequence
<220>
   <223> DcysMR
<400> 17
   cccgccccct ggctaaaatg ctcttcccca aacaccccgg tagaaaggta gcgatcgcca 60
   cgatcgcaga tgatcgccac catatgaata tcctccttag 100
<210> 18
   <211> 100
   <212> DNA
   <213> Artificial sequence
<220>
   <223> DcySMF
<400> 18
   agtacattag aacaaacaat aggcaatacg cctctggtga agttgcagcg aatggggccg 60
   gataacggca gtgaagtgtg tgtaggctgg agctgcttcg 100
<210> 19
   <211> 30
   <212> DNA
   <213> Artificial Sequence
<220>
   <223> cysKR
<400> 19
   tttttaacag acgcgacgca cgaagagcgc 30
<210> 20
   <211> 30
   <212> DNA
   <213> Artificial Sequence
<220>
   <223> cysKR
<400> 20
   ggcgcgacgg cgatgtgggt cgattgctat 30
<210> 21
   <211> 30
   <212> DNA
   <213> Artificial sequence
<220>
   <223> cysMR
<400> 21
   ggggtgacgg tcaggactca ccaatacttc 30
<210> 22
   <211> 30
   <212> DNA
   <213> Artificial sequence
<220>
   <223> cysMF
<400> 22
   gcgcgcatcg ctggccgctg ggctacacac 30
<210> 23
   <211> 74
   <212> DNA
   <213> Artificial sequence
<220>
   <223> MetYR
<400> 23
   ttagagctgt tgacaattaa tcatccggct cgtataatgt gtggaataaa aactcttaag 60
   gacctccaaa tgcc 74
<210> 24
   <211> 30
   <212> DNA
   <213> Artificial sequence
<220>
   <223> MetYF
<400> 24
   gctctgtcta gtctagtttg cattctcacg 30
<210> 25
   <211> 100
   <212> DNA
   <213> Artificial sequence
<220>
   <223> DudhAR
<400> 25
   cccagaatct cttttgtttc ccgatggaac aaaattttca gcgtgcccac gttcatgccg 60
   acgatttgtg cgcgtgccag tgtaggctgg agctgcttcg 100
<210> 26
   <211> 100
   <212> DNA
   <213> Artificial Sequence
<220>
   <223> DudhAF
<400> 26
   ggtgcgcgcg tcgcagttat cgagcgttat caaaatgttg gcggcggttg cacccactgg 60
   ggcaccatcc cgtcgaaagc catatgaata tcctccttag 100
<210> 27
   <211> 30
   <212> DNA
   <213> Artificial sequence
<220>
   <223> UdhAR
<400> 27
   gcgggatcac tttactgcca gcgctggctg 30
<210> 28
   <211> 30
   <212> DNA
   <213> Artificial Sequence
<220>
   <223> udhAF
<400> 28
   ggccgctcag gatatagcca gataaatgac 30
<210> 29
   <211> 100
   <212> DNA
   <213> Artificial sequence
<220>
   <223> DpgiR
<400> 29
   gcgccacgct ttatagcggt taatcagacc attggtcgag ctatcgtggc tgctgatttc 60
   tttatcatct ttcagctctg catatgaata tcctccttag 100
<210> 30
   <211> 100
   <212> DNA
   <213> Artificial Sequence
<220>
   <223> DpgiF
<400> 30
   ccaacgcaga ccgctgcctg gcaggcacta cagaaacact tcgatgaaat gaaagacgtt 60
   acgatcgccg atctttttgc tgtaggctgg agctgcttcg 100
<210> 31
   <211> 30
   <212> DNA
   <213> Artificial Sequence
<220>
   <223> PgiR
<400> 31
   cggtatgatt tccgttaaat tacagacaag 30
<210> 32
   <211> 30
   <212> DNA
   <213> Artificial Sequence
<220>
   <223> PgiF
<400> 32
   gcggggcggt tgtcaacgat ggggtcatgc 30
<210> 33
   <211> 100
   <212> DNA
   <213> Artificial sequence
<220>
   <223> DpfkAR
<400> 33
   ttcgcgcagt ccagccagtc acctttgaac ggacgcttca tgttttcgat agcgtcgatg 60
   atgtcgtggt gaaccagctg catatgaata tcctccttag 100
<210> 34
   <211> 100
   <212> DNA
   <213> Artificial Sequence
<220>
   <223> DpfkAF
<400> 34
   ggtgtgttga caagcggcgg tgatgcgcca ggcatgaacg ccgcaattcg cggggttgtt 60
   cgttctgcgc tgacagaagg tgtaggctgg agctgcttcg 100
<210> 35
   <211> 27
   <212> DNA
   <213> Artificial sequence
<220>
   <223> PfkAR
<400> 35
   ccctacgccc cacttgttca tcgcccg 27
<210> 36
   <211> 27
   <212> DNA
   <213> Artificial sequence
<220>
   <223> PfkAF
<400> 36
   cgcacgcggc agtcagggcc gacccgc 27
<210> 37
   <211> 100
   <212> DNA
   <213> Artificial sequence
<220>
   <223> DpfkBR
<400> 37
   gcgggaaagg taagcgtaaa ttttttgcgt atcgtcatgg gagcacagac gtgttccctg 60
   attgagtgtg gctgcactcc catatgaata tcctccttag 100
<210> 38
   <211> 99
   <212> DNA
   <213> Artificial Sequence
<220>
   <223> DpfkBF
<400> 38
   gcgccctctc tcgatagcgc aacaattacc ccgcaaattt atcccgaagg aaaactgcgc 60
   tgtaccgcac cggtgttcgt gtaggctgga gctgcttcg 99
<210> 39
   <211> 26
   <212> DNA
   <213> Artificial sequence
<220>
   <223> PfkBR
<400> 39
   gccggttgca ctttgggtaa gccccg 26
<210> 40
   <211> 30
   <212> DNA
   <213> Artificial sequence
<220>
   <223> PfkBF
<400> 40
   tggcaggatc atccatgaca gtaaaaacgg 30
<210> 41
   <211> 29
   <212> DNA
   <213> Artificial Sequence
<220>
   <223> YveDR
<400> 41
   cgtgaattct tattcatcaa ttctaataa 29
<210> 42
   <211> 32
   <212> DNA
   <213> Artificial Sequence
<220>
   <223> YueDF
<400> 42
   acgttcatga gatacgttat cataacagga ac 32

## Description

La présente invention concerne un nouveau procédé de préparation de microorganismes évolués permettant la création ou la modification de voies métaboliques, les souches de microorganismes évolués ainsi obtenus, les gènes évolués codant pour des protéines évoluées susceptibles d'être obtenues par le procédé selon l'invention et l'utilisation desdits microorganismes, gènes ou protéines évolués dans un procédé de biotransformation.

La préparation de microorganismes à propriétés modifiées est un processus largement répandu. Il s'agit soit de faire évoluer des microorganismes en les faisant croître sur un milieu de croissance avec un élément de pression de sélection, de manière à sélectionner les microorganismes capables de résister à cette pression, soit à introduire un ou plusieurs gènes hétérologues par des techniques aujourd'hui largement répandues de génie génétique, afin de conférer aux microorganismes des nouveaux caractères phénotypiques associés à l'expression du ou desdits gènes hétérologues. L'évolution peut être favorisée par l'utilisation d'agents mutagènes bien connus de l'homme du métier. Des mutations aléatoires peuvent égalemenent être introduites par les techniques classiques de « error-prone PCR » ou mutagenèse par saturation. Chirumamilla et al. (Molecular and Cellular Biochemistry 224 :159-168, 2001) décrivent un procédé d'évolution de protéines *in vitro* basé sur de la mutagenèse aléatoire réalisée par ces techniques.

Des techniques d'évolution par croissance sous pression de sélection en supprimant un gène nécessaire à la transformation d'un composant présent dans le milieu de culture et au moyen d'un agent mutagène sont décrites notamment dans WO 02/083892 ou WO 03/004656. Une étude de réversion d'une souche de *E. coli* auxotrophe pour le L-tryptophane dans laquelle la fonction de synthèse est restaurée est aussi décrite dans Allen & Yanofsky (Genetics, vol. 48, 1963-08, pp 1065-1083). Par ailleurs, un procédé d'évolution permettant de changer la spécificité pour un substrat d'une enzyme a été décrit par Hall. BG. (Biochemistry, 1981, 20, p 4042-4049). En particulier, BG. Hall décrit la sélection de mutants spontanés capables de survivre sur un milieu contenant du lactose, alors que l'enzyme *ebg* betagalactosidase de ces souches ne permet pas habituellement le métabolisme du lactose. D'autres procédés permettent l'évolution de microorganismes (Mortlock RP., Ann. Rev. Microbiol. 1982. 36 :259-84). Ce procédé spécifique conduit à l'obtention de microorganismes capables d'utiliser un certain substrat, mais sans modification des voies métaboliques de biosynthèse.

### Brève description de l'invention

La présente invention concerne un nouveau procédé de préparation de microorganismes évolués d*'Escherichia coli,* permettant une modification de la voie de biosynthèse de la méthionine, caractérisé en ce qu'il consiste en les étapes suivantes :
a) Obtention d'un microorganisme modifié par inactivation de l'activité methionine synthase native dans les cellules d'un microorganisme initial de manière à inhiber la production de méthionine lorsque le microorganisme est cultivé sur un milieu défini affectant ainsi la capacité de croissance du microorganisme, la modification comprenant la délétion du gène metE,
b) Culture du microorganisme modifié précédemment obtenu sur ledit milieu défini pour le faire évoluer, en présence de methylmercaptan ou de l'un de ses sels n'ayant pas de propriété mutagène, ledit milieu défini étant dépourvu de méthionine, S-adenosylmethionine, homocysteine et cystathionine, et
c) Sélection des cellules de microorganismes modifiés capables de se développer sur le milieu défini, en présence de methylmercaptan, les microorganismes évolués étant des microorganismes modifiés comprenant une cystathionine γ-synthase endogène à activité methionine synthase améliorée.

La présente invention concerne aussi un procédé d'obtention d'un gène évolué codant pour une cystathionine γ-synthase endogène à activité methionine synthase améliorée, ledit procédé comprenant la préparation d'un microorganisme évolué, ledit microorganisme évolué étant une souche E. coli K183 déposée sous le numéro 1-3005 à la CNCM, comprenant une cystathionine γ-synthase endogène à activité methionine synthase améliorée, et comprenant une étape d) d'isolation du gène évolué codant pour ladite protéine évoluée.

L'invention concerne également une souche E. coli K183 déposée sous le numéro I-3003 à la CNCM, capable de se développer sur ledit milieu défini en présence de methylmercaptan ou de l'un de ses sels n'ayant pas de propriété mutagène, comprenant une cystathionine γ-synthase endogène à activité methionine synthase améliorée.

L'invention concerne également un procédé de préparation de méthionine comprenant les étapes suivantes :
a) mise en culture d'une souche E.coli K183 déposée sous le numéro I-3005 à la CNCM, en présence de methylmercaptan et d'une source de carbone simple ;
b) extraction de la méthionine et le cas échéant purification.

### Définitions

Par « microorganisme évolué », on entend selon l'invention un microorganisme obtenu par sélection d'un microorganisme modifié. Le microorganisme évolué présente au moins une différence par rapport au microorganisme modifié, cette différence correspond par exemple à une amélioration d'une caractéristique enzymatique ou encore à la création d'une nouvelle voie métabolique.

Par «voie métabolique », on entend selon l'invention une ou plusieurs réactions enzymatiques dont la succession permet de produire une molécule (produit) différente de la molécule initiale (substrat).

Par « modification », on entend selon l'invention une altération, en particulier une délétion, d'au moins un gène et/ou de sa séquence promotrice, le gène codant pour une enzyme.

Par « métabolite », on entend selon l'invention une molécule synthétisée et/ou transformée par le microorganisme.

Par « milieu défini », on entend selon l'invention un milieu de composition moléculaire connue, adapté à la croissance du microorganisme. Le milieu défini est substantiellement exempt du ou des métabolites dont on inhibe la production en réalisant la modification.

Par « co-substrat », on entend selon l'invention une molécule carbonée ou non, différente du substrat, qui est impliqué dans une réaction et donnant un ou plusieurs atomes au substrat afin de former le produit. Le co-substrat n'a pas de propriété mutagène reconnue.

Par « sélection », on entend selon l'invention un procédé de culture permettant de sélectionner les microorganismes ayant évolué de telle sorte que la modification n'affecte plus la croissance. Une application préférée est un procédé de culture en continu, conduit en appliquant des taux de dilution croissants de telle sorte de ne conserver dans le milieu de culture que les microorganismes ayant un taux de croissance égal ou supérieur au taux de dilution imposé.

Par « gène évolué », on entend selon l'invention une succession d'acides nucléiques (pris parmi A,T,G ou C) délimité par un codon stop (TAA, TAG, TGA) en phase et ayant, à l'issue de la sélection, au moins un acide nucléique différent par rapport à la séquence initiale de telle sorte que la protéine codée par ce gène évolué présente au moins un acide aminé différent par rapport à la protéine codée par le gène initial.

Par « gène hétérologue », on entend selon l'invention une succession d'acides nucléiques, délimitée par un codon start (ATG ou GTG) et un codon stop (TAA, TAG, TGA) en phase, dénommée séquence codante et issue d'un organisme différent de celui utilisé pour réaliser l'évolution et/ou la production.

Par « protéine évoluée », on entend selon l'invention une succession d'acides aminés (séquence protéique) ayant, à l'issue de la sélection, au moins un acide aminé différent par rapport à la séquence protéique initiale.

Par « protéine hétérologue », on entend selon l'invention une protéine issue de la traduction d'un gène hétérologue.

Les gènes et protéines peuvent être identifiées par leurs séquences primaires, mais également par homologies de séquences ou alignements qui définissent des groupes de protéines.

Les PFAM (Protein families database of alignments and Hidden Markov Models ; http:-//www.sanger.ac.uk/Software/Pfam/) représentent une large collection d'alignements de sequences protéiques. Chaque PFAM permet de visualiser des alignements multiples, de voir des domaines protéiques, d'évaluer la répartition entre les organismes, d'avoir accès à d'autres bases de données, de visualiser des structures connues de protéines.

Les COGs (Clusters of Orthologous Groups of proteins ; http://www.ncbi.nlm.nih.gov/COG/) sont obtenus en comparant les séquences protéiques issus de 43 génomes complètement séquencés représentant 30 lignées phylogénétiques majeurs. Chaque COG est défini à partir d'au moins trois lignées ce qui permet ainsi d'identifier des domaines conservés anciens.

Les moyens d'identification des séquences homologues et de leurs pourcentages d'homologie sont bien connus de l'homme du métier, comprenant notamment les programmes BLAST qui peuvent être utilisé à partir du site http://www.ncbi.nlm.nih.gov/BLAST/ avec les paramètres indiqués par défaut sur ce site. Les séquences obtenues peuvent alors être exploitées (e.g. alignées) en utilisant par exemple les programmes CLUSTALW (http://www.ebi.ac.uk/clustalw/) ou MULTALIN (http://prodes.toulouse.inra.fr/multalin/cgi-bin/multalin.pl), avec les paramètres indiqués par défaut sur ces sites.

En utilisant les références données sur GenBank pour les gènes qui sont connus, l'homme du métier est capable de déterminer les gènes équivalents dans d'autres organismes, souches bactériennes, levures, champignons, mammifères, plantes, etc. Ce travail de routine est avantageusement effectué en utilisant les séquences consensus pouvant être déterminées en réalisant des alignements de séquences avec des gènes issus d'autres microorganismes, et en dessinant des sondes dégénérées permettant de cloner le gène correspondant dans un autre organisme. Ces techniques de routine de biologie moléculaire sont bien connues dans l'art et sont décrites par exemple dans Sambrook et al. (1989 Molecular cloning : a laboratory manual. 2nd Ed. Cold Spring Harbor Lab., Cold Spring Harbor, New York.).

Les gènes susceptibles d'être délétés ou surexprimés pour les souches évoluées selon l'invention sont définis principalement par l'emploi de la dénomination du gène de *E. coli.* Cependant, cet emploi a une signification plus générale selon l'invention et englobe les gènes correspondants d'autres microorganismes. En effet en utilisant les références GenBank des gènes d'*E*. *coli,* l'homme du métier est capable de déterminer les gènes équivalents dans d'autres souches bactériennes qu'*E. coli.*

Par « nouvelle voie métabolique », on entend selon l'invention une ou plusieurs réactions enzymatiques, dont la succession permet de produire une molécule, qui, après l'étape de sélection du microorganisme évolué, se révèlent différentes des activités enzymatiques du microorganisme non évolué. La différence pouvant résider dans le type de réaction catalysée ou bien dans ses caractéristiques cinétiques (Km, Vmax, Ki, ...). Une nouvelle voie enzymatique permet de produire une molécule, différente ou non du produit initial, à partir d'un substrat différent ou non du substrat initial.

Par « forme appropriée », on entend selon l'invention une succession d'acide nucléique, délimitée par un codon start (ATG ou GTG) et un codon stop (TAA, TAG, TGA) en phase, dénommée séquence codante, ou une partie de cette séquence codante, sous le contrôle d'éléments de régulation nécessaires à son expression dans le microorganisme dans lequel le gène hétérologue sera exprimé. Ces éléments de régulations sont bien connus de l'homme du métier, et comprennent des séquences de régulation promotrice, ou promoteurs, en particulier des promoteurs dits promoteurs forts constitutifs chez les microorganismes. De préférence, le promoteurs constitutif est choisi parmi pTAC-O, pLAC-O, pTRC-O, des promoteurs forts pour lesquels l'opérateur lac a été délété pour les rendre constitutifs, pTHLA.

Par « microorganisme initial » on entend un microorganisme avant réalisation de toute modification, mutation ou évolution.

Par « microorganisme de production », on entend selon l'invention un microorganisme évolué ou un microorganisme optimisé dans le lequel a été introduit la nouvelle voie métabolique issue d'un microorganisme évolué.

Par « microorganisme modifié » on entend un microorganisme obtenu après réalisation de modifications maîtrisées par l'homme et ne résultant pas d'un processus d'évolution. On citera à titre d'exemple de modification, la mutation dirigée ou la délétion d'un gène, la modification dirigée d'un promoteur.

Par « milieu de culture approprié pour la production de la protéine évoluée », on entend selon l'invention soit un milieu de composition défini, soit un milieu complexe, soit un milieu partiellement défini. Le milieu complexe est réalisé à partir soit d'un hydrolysat végétal, de microorganisme ou animal ; sa composition peut-être connue en réalisant des analyses, il est cependant rare de pouvoir réaliser une analyse exhaustive de ce type de milieu. Un milieu partiellement défini est un milieu défini auquel on rajoute un milieu complexe.

Par « procédé de biotransformation » on entend selon l'invention un procédé de transformation d'une molécule A en une molécule B en utilisant une ou plusieurs enzymes, contenues ou non dans un ou plusieurs microorganismes. On distingue trois types de procédés de biotransformation : la bioconversion, la fermentation et la biocatalyse. Dans le cas de la bioconversion, la ou les enzymes sont contenues dans un ou plusieurs microorganismes, ceux-ci sont cultivés sur un milieu approprié puis la molécule A et éventuellement un ou plusieurs co-substrats sont apportés pour être transformés en B. Dans le cas de la fermentation, la ou les enzymes sont contenues dans un ou plusieurs microorganismes, ceux-ci sont cultivés sur un milieu approprié permettant au(x) microorganisme de synthétiser la molécule A ; le milieu approprié peut contenir des co-substrats. Dans le cas de la biocatalyse, la ou les enzymes ne sont pas contenues dans des cellules mais sont entourées par un milieu adéquat apportant la molécule A ainsi que les co-substrats nécessaires à la biotransformation.

Les méthodes d'isolation de gènes sont bien connues de l'homme du métier, décrites notamment dans Sambrook et al, (1989 Molecular cloning : a laboratory manual. 2nd Ed. Cold Spring Harbor Lab., Cold Spring Harbor, New York), Ausubel et al., 1987 (Current Protocols in Molecular Biology, John Willey and Sons, New York); Maniatis et al., 1982, (Molecular Cloning : a laboratory manual. Cold Spring Harbor Laboratory, Cold Sring Harbor, New York). Ces méthodes permettent de localiser, de copier ou d'extraire le gène, afin de l'introduire dans un nouvel organisme. Cette dernière étape pouvant être précédée d'une étape d'incorporation du gène dans un polynucléotides préalablement à son introduction dans le microorganisme.

Les méthodes de purification d'une protéine sont bien connues de l'homme du métier, décrites notamment dans Coligan et al., 1997 (Current Protocole in Protein Science, John Wiley & Sons, Inc). Elles permettent d'identifier la protéine recherchée dans un extrait protéique, fractionné ou non. La protéine peut ensuite être purifiée, ce qui se traduit par une augmentation de son activité spécifique, dans le cas particulier d'une enzyme. Enfin ces techniques permettent éventuellement d'immobiliser la protéine sur un support (e.g. résine).

Par « délétion », on entend selon l'invention une suppression de l'activité du gène « délété ». Cette suppression peut être une inactivation du produit d'expression du gène concerné par un moyen approprié, ou bien l'inhibition de l'expression du gène concerné, ou encore la délétion d'au moins une partie du gène concerné de manière soit que son expression n'ait pas lieu (par exemple délétion d'une partie ou de l'ensemble de la région promotrice nécessaire à son expression) soit que le produit d'expression ait perdu sa fonction (par exemple délétion dans la partie codante du gène concerné). De manière préférentielle, la délétion d'un gène comprend la suppression de l'essentiel dudit gène, et le cas échéant son remplacement par un gène marqueur de sélection permettant de faciliter l'identification, l'isolement et la purification des souches évoluées selon l'invention.

Par « substrat » on entend un métabolite qui peut être transformé par l'action d'une enzyme, éventuellement en présence d'un co-substrat.

### Description détaillée de l'invention

Par souche de microorganismes, on entend selon l'invention un ensemble de microorganismes d'une même espèce comprenant au moins un microorganisme de ladite espèce. Ainsi, les caractéristiques décrites pour la souche s'appliquent à chacun des microorganismes de ladite souche. De même, les caractéristiques décrites pour l'un des microorganismes de la souche s'appliqueront à l'ensemble desdits microorganismes la composant.

Les microorganismes optimisés selon l'invention sont choisis parmi les

Pour préparer lesdit microorganismes modifiés, il peut être avantageux d'atténuer, en particulier de déléter, d'autres gènes associés ou non à la voie métabolique à modifier afin de favoriser l'évolution du microorganisme.

Pour préparer lesdit microorganismes modifiés, il peut être également avantageux de favoriser, en particulier de surexprimer, d'autres gènes, hétérologues ou non, associés ou non à la voie métabolique à modifier afin de favoriser l'évolution du microorganisme.

La surexpression d'un gène peut être effectuée par changement du promoteur de ce gène *in situ,* par un promoteur fort ou inductible. De façon alternative, on introduit, dans la cellule, un plasmide réplicatif (simple ou multicopies) dans lequel le gène que l'on désire surexprimer est sous le contrôle du promoteur adéquat.

De telles modifications seront décidées au cas par cas selon le choix de la voie métabolique à modifier. Elles sont notamment décrites au cas par cas pour les voies métaboliques particulières exposées ci-après.

L'homme du métier connaît les protocoles permettant de modifier le caractère génétique de microorganismes.

L'inactivation d'un gène se fait préférentiellement par recombinaison homologue. (Datsenko, K.A. ; Wanner, B.L. (2000) One-step inactivation of chromosomal genes in Escherichia coli K-12 using PCR products. Proc. Natl, Acad. Sci. USA 97 : 6640-6645). Le principe d'un protocole en est rappelé briévement : on introduit dans la cellule un fragment linéaire, obtenu *in vitro,* comprenant les deux régions flanquant le gène, et au moins un gène de sélection entre ces deux régions (généralement un gène de résistance à un antibiotique), ledit fragment linéaire présentant donc un gène inactivé. On sélectionne les cellules ayant subi un événement de recombinaison et ayant intégré le fragment introduit par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaisons.

L'inactivation d'un gène chez *S. cerevisiae* se fait préférentiellement par recombinaison homologue (Baudin et al., Nucl. Acids Res. 21, 3329-3330, 1993 ; Wach et al., Yeast 10, 1793-1808, 1994; Brachmann et al., Yeast. 14 : 115-32, 1998).

Les microorganismes de productions sont choisis également parmi les souches d'E. coli. Il peut s'agir des microorganismes évolués obtenus par le procédé d'évolution selon l'invention, ou encore de microorganismes optimisés pour la production du métabolite recherché, dans lesquels on aura introduit au moins un gène évolué selon l'invention.

Selon l'invention, les termes « culture » et « fermentation » sont employés indifféremment pour désigner la croissance du microorganisme sur un milieu de culture approprié comprenant une source de carbone simple.

Par source de carbone simple, selon la présente invention, on entend des sources de carbone utilisables par l'homme du métier pour la croissance normale d'un microorganisme, d'une bactérie en particulier. On entend désigner notamment les différents sucres assimilables, tels le glucose, le galactose, le saccharose, le lactose ou les mélasses, ou les sous-produits de ces sucres. Une source de carbone simple tout particulièrement préférée est le glucose. Une autre source de carbone simple préférée est le saccharose.

La définition des conditions de culture des microorganismes selon l'invention est à la portée de l'homme du métier. On fermente notamment les bactéries à une température comprise entre 20°C et 55°C, de préférence entre 25°C et 40°C, plus particulièrement d'environ 37°C pour *E. coli.*

La fermentation est généralement conduite en fermenteurs avec un milieu minéral de culture de composition connu défini et adapté en fonction des bactéries utilisées, contenant au moins une source de carbone simple et le cas échéant un co-substrat nécessaire à la production du métabotite.

En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992 ; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96).

La voie métabolique destinée à être évoluée voie de biosynthèse de la méthionine,

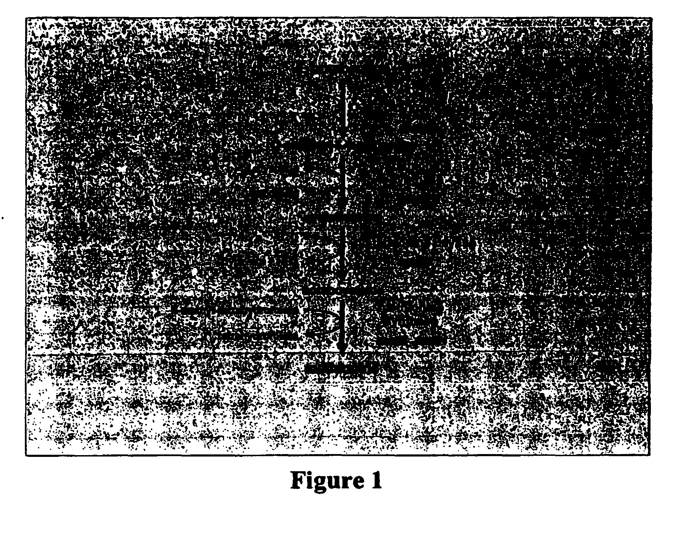

L'application de l'invention peut se faire par exemple sur la voie de biosynthèse de la méthionine (figure 1) et permet d'obtenir des souches de microorganismes, en particulier des bactéries, notamment *E. colt* et les corynébactéries, produisant de l'acide 2-Amino-4-(alkylimercapto)butyrique, en particulier de la L-méthionine (acide 2-Amino-4-(méthylmercapto)butyrique) par métabolisme d'une source de carbone simple et d'une source de soufre, en particulier le methylmercaptan (CH3SH), le sulfure d'hydrogène (H2S) ou leurs sels. La source de soufre H2S peut aussi être introduite dans le milieu de culture sous la forme de sulfate. L'invention concerne également la souche de microorganisme, les enzymes améliorées et à leurs séquences codantes. L'invention concerne enfin un procédé de préparation de la méthionine par culture de ladite souche de microorganisme.

La DL-Methionine est produite industriellement par synthèse chimique. Le méthyl-mercaptan réagit avec l'acroléine pour produire le β-méthyl thiopropionaldéhyde, qui réagit avec l'hydrogène cyanide pour produire l'α-hydroxy-γ-méthyl-thio-butyro-nitrile, Après traitement avec l'ammoniac, et une hydrolyse, on obtient la méthionine.

Tous les producteurs industriels de la DL-methionine utilisent les mêmes matières premières à savoir l'acroléine, le méthane thiol (méthyl-mercaptan), l'hydrogène cyanide et l'ammoniac ou l'ammonium carbonate. Le procédé de synthèse du mélange racémique peut être conduit en batch ou en continu.

Un procédé industriel combine à la synthèse chimique de la biocatalyse en utilisant l'amino acylase, enzyme produite par *Aspergillus oryzas* pour obtenir uniquement la L-méthionine à partir de la DL-méthionine.

Les brevets US 6,379,934 et BP 1 055 730 se rapportent à la production d'acides aminés en utilisant une souche de bactéries corynéformes, dans laquelle le gène *acc*BC est amplifié. La méthionine est mentionnée, mais seule la préparation de L-lysine est exemplifiée.

toutefois, la synthèse d'acides aminés soufrés en culture de microorganisme reste difficile à mettre en oeuvre à des niveaux susceptibles de conduire à une exploitation industrielle, notamment du fait de la complexité de leurs voies de biosynthèse et de nombreux mécanismes de régulation. En effet, le métabolisme de la méthionine, est fortement régulé, la régulation de son métabolisme se faisant à plusieurs niveaux (Weissbach et al., 1991, Mol. Microbiol., 5, 1593-1597) : métabolisme carboné pour la fabrication de la L-sérine à partir du a glycerate3P, de la L-homosérine à partir de l'aspartate et de l'acétyl-CoA métabolisme du soufre pour la fabrication de la L-cystéine à partir de la L-sérine, de l'acétyl-CoA et du sulfate du milieu de culture

synthèse de la méthionine (Fig. 1) à partir de la L-homosérine, de la cystéine et d'acétyl-CoA ou succinyl-CoA. ou

La demande WO 93/17112 décrit les différentes enzymes impliquées dans la biosynthése de la méthionine à partir de l'acide L-aspartique dans divers organismes. Cette demande de brevet décrit également l'introduction dans un microorganisme de plusieurs gènes exogènes agissant de manière séquentielle pour la synthèse de la méthionine, employant du méthyl-mercaptan ou du sulfure d'hydrogène comme source de soufre.

La présente invention se rapporte à des souches de *E. coli* produisant de l'acide 2-amino-4-(alkylmercapto)butyrique de formule générale (I)

R-S-(CH₂)₂-CHNH₂-COOH (I)

dans laquelle R représente un radical alkyle linéaire ou ramifié comprenant de 1 à 18 atomes de carbones, éventuellement substitué par un ou des groupe(s) hydroxy, ou un radical aryle ou hétéroaryle comprenant un ou plusieurs atomes d'azote ou de soufre dans le cycle hétéroaromatique, sélectionné parmi les groupes phényle, pyridyle, pyrolyle, pyrazolyle, triazolyle, tetrazolyle, thiazolyle, ou thienyle,
par métabolisme d'une source de carbone simple et d'une source de soufre comprenant un composé de formule générale (II) :

R'-SH (II)

dans laquelle R' représente un atome d'hydrogène ou R, R étant défini précédemment, et ses sels physiologiquement acceptables,

les dites souches présentant au moins un gène codant pour une enzyme à activité « méthionine synthase » modifiée.

Par enzyme à activité « méthionine synthase » modifiée, on entend selon l'invention toute enzyme impliquée dans la biosynthèse des acides 2-amino-4-(alkylmercapto)butyriques de formule générale (I) par conversion d'un substrat dérivé de formule générale (III)

R"-O-(CH₂)₂-CHNH₂-COOH (III)

dans laquelle R" représente un radical acyle de préférence choisi parmi le radical succinyle ou le radical acétyle,
par conversion directe du substrat en acide de formule générale (I).

Les enzymes à activité « méthionine synthase » modifiée sont des enzymes modifiées par rapport aux enzymes natives pour leur permettre de réaliser de manière préférentielle la conversion directe du substrat de formule générale (III) en acide de formule générale (I) lieu de la réaction catalysée par l'enzyme native. Cette modification, appelée encore mutation, consiste essentiellement en ce que l'enzyme modifiée ait une plus grande affinité pour le composé soufré de formule générale (II) que pour son co-substrat naturel.

Par source de carbone simple, selon la présente invention, on entend des sources de carbone utilisables par l'homme du métier pour la croissance normale d'un microorganisme, d'une bactérie en particulier. On entend désigner notamment les différents sucres assimilables, tels le glucose, le galactose, le saccharose, le lactose ou les mélasses, ou les sous-produits de ces sucres. Une source de carbone simple tout particulièrement préférée est le glucose. Une autre source de carbone simple préférée est le saccharose.

Par sel physiologiquement acceptable, on entend selon l'invention les sels du composé de formule générale (I) qui n'affectent pas le métabolisme et la capacité de croissance de la souche de microorganisme selon l'invention, notamment les sels de métaux alcalins comme le sodium.

Selon un mode préférentiel de réalisation de invention, R représente un radical alkyle linéaire ou ramifié comprenant de 1 à 4 atomes de carbone, en particulier choisi parmi les radicaux méthyle, éthyle, n-propyle, i-propyle, n-butyle, i-butyle ou t-butyle. De manière plus préférentielle, R représente le radical méthyle.

L'acide 2-amino-4-(alkylmercapto)butyrique de formule générale (I) obtenu est la L-méthionine.

L'utilisation d'une source de carbone simple et d'un composé soufré de formule (II) pour la production d'acide de formule (I) par bioconversion présente *a priori* plusieurs avantages, notamment :
la synthèse de l'acide (I), comme la méthionine, en une étapes à partie à partir d'O-acyl-L-homosérine, devient indépendante de la synthèse de la cystéine voire également du cycle du tétrahydrofolate ;
- les composés soufrés de formule (II), comme le méthyl-mercaptan, qui sont des matières premières généralement toxiques issues de la pétrochimie, peuvent être valorisés dans la synthèse d'acides aminés à haute valeur ajoutée.

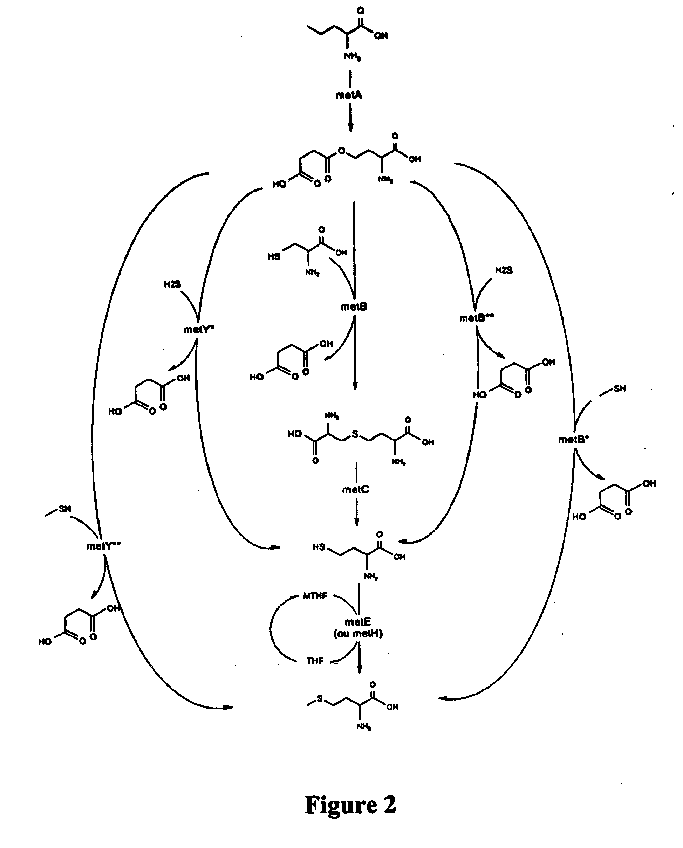

L'invention est basée sur le fait que l'on peut obtenir, de façon dirigée, une modification de l'activité « méthionine-synthase » de la cystationine-γ-synthase (EC 4.2.99.9 ; GenBank AAN83320, ou AAA24167) en présence de méthyl-mercaptan, Cette enzyme de la voie de biosynthése de méthionine, codée par le gène *met*B chez *E. coli* (Fig. 2) et C. *glutamicum,* présente une activité pour un large spectre de substrats (Flavin, M. ; Slaughter, C. (1967) Enzymatic synthesis of homoserine or methionine directly from O-succinyl-homoserine.Biochim. Biophys. Acta 132: 400-405).

L'invention concerne donc également un procédé de préparation des souches selon l'invention et leur utilisation dans un procédé de préparation de la L-méthionine.

Le procédé de préparation de souches selon l'invention consiste à obtenir, à partir d'une souche bactérienne initiale, une souche bactérienne génétiquement modifiée présentant au moins une modification dans un gène codant pour une enzyme à activité « méthionine synthase », ledit procédé comprenant une étape consistant à soumettre ladite souche bactérienne initiale à une pression de sélection en présence du composé de formule (II) défini ci-dessus, afin de diriger une évolution dudit gène codant pour ladite enzyme à activité « méthionine synthase » dans ladite souche bactérienne, vers un gène codant pour une enzyme à activité «méthionine synthase » modifiée par rapport à ladite souche bactérienne initiale.

Une activité « méthionine synthase » est améliorée dans la souche (A) de microorganisme par rapport à la souche (I) initiale lorsque la production de méthionine dans les mêmes conditions de culture (dans un milieu contenant une quantité efficace de dérivé soufré de formule (II)) est supérieure pour la souche (A) que pour la souche (I). Cette amélioration est préférentiellement observée par étude de la quantité de méthionine produite. Dans certains cas, on peut observer cette amélioration par l'augmentation du taux de croissance de la bactérie (A) par rapport au taux de croissance de la bactérie (I), dans un milieu minimum ne contenant pas de méthionine.

La présente invention concerne également la souche E. coli K183 déposée sous le numéro E-3005 à la CNCH.

L'enzyme à activité « méthionine synthase » modifiée permet la conversion directe du substrat de formule générale (III) en acide de formule générale (I). Dans ce cas, la source de soufre est un composé de formule générale (II) pour laquelle R' représente le radical R définit précédemment.

L'enzyme à activité « méthionine synthase » modifiée est la cystathionine-γ-synthases

Pour les cystathionine-γ-synthases modifiées définies précédemment et ci-dessous, le substrat est avantageusement la O-acétyl-L-homosérine ou la O-succinyl-L-homosérine, de préférence la O-succinyl-L-homosérine.

Pour cette enzyme, la modification consisté essentiellement en une mutation de manière à ce que la transformation du substrat de formule générale (III) se fasse de manière préférentielle avec le composé de formule générale (II) plutôt qu' avec la L-cystéine.

La mutation des enzymes est obtenue par la mise en oeuvre du procédé de préparation de souches à activité « méthionine synthase » améliorée selon l'invention par culture sous pression de sélection en présence du composé de formule générale (II).

Dans ce cas, le gène comprenant la séquence codant pour la cystathionine-γ-synthase est
- soit un gène natif, présent dans le génome de la souche initiale (I) où il est exprimé pour permettre la traduction de l'enzyme correspondante,
- soit un gène hétérologue comprenant une séquence codant pour une cystathionine-γ-synthase sous le contrôle d'éléments de régulation permettant son expression et sa traduction dans la souche initiale (I) où il aura été introduit. La mutation peut également être obtenue par mutagénèse dirigée,
- soit directement sur le gène natif présent naturellement dans la souche initiale (I), notamment par recombinaison homologue,
- soit par des techniques usuelles de mutagenèse dirigée sur des séquences codant pour une cystathionine-γ-synthase introduite ensuite dans la souche initiale (I) sous le contrôle d'éléments de régulation permettant son expression et sa traduction dans ladite souche initiale (I) où il aura été introduit.

De tels éléments de régulation sont bien connus de l'homme du métier, et comprennent des séquences de régulation promotrice, ou promoteurs, en particulier des promoteurs dits promoteurs forts constitutifs chez les microorganismes. De préférence, le promoteur fort constitutif est choisi parmi pTAC-O, pLAC-O, pTRC-O, pTHLA, promoteurs forts pour lesquels l'opérateur lac a été délété pour les rendre constitutifs.

De manière avantageuse, les cystathionine-γ-synthases « initiales » ou à activité « méthionine synthase » non modifiée sont sélectionnées parmi les cystathionine-γ-synthases correspondent au PFAM référence PF01053 et au COG référence CPG0626.

De manière préférentielle, la cystathionine-γ-synthase est la cystathionine-γ-synthase de E. coli K12, représentée sur la SEQ ID NO ; 6, et les séquences homologues de cette séquence présentant une activité cystathionine-γ-synthase et comprenant au moins 80% d'homologie, préférentiellement 90% d'homologie, plus préférentiellement 95% d'homologie avec la séquence d'acides aminés de la SEQ ID NO 6.

Les moyens d'identification des séquences homologues et de leur pourcentages d'homologie sont bien connus de l'homme du métier, comprenant notamment le programme BLAST, et notamment le programme BLASTP, qui peut être utilisé à partir du site http://www.ncbi.nlm.nih.gov/BLAST/ avec les paramètres indiqués par défaut sur ce site.

Selon un mode préférentiel de réalisation de l'invention, la cystathionine-γ-synthase initiale, avant modification, comprend une séquence d'acide aminés ci-dessous dans sa partie C-terminale (zone conservée 1)

X1-X2-X3-L-G-X4-X5-X6-X7-X8-X9

dans laquelle
X1 représente A,G,S, de preference A
X2 représente E,V,P,T, de preference E
X3 représente S,T,N, de preference S
X4 représente G,D,A,H,T, de preference G
X5 représente V,A,T,H,N, de preference V
X6 représente E,R,K,F, de preference E
X7 représente S,T, de preference S
X8 représente L,I,V,A, de preference L et
X9 représente I,V,A,T, de preference I.

De manière préférentielle, la cystathionine-γ-synthase à activité « méthionine synthase » modifiée, selon l'invention comprend la séquence d'acides aminés suivante dans sa partie C-terminale :

A-E-S-L-G-G-V-E-S

De manière avantageuse, la cystathionine γ-synthase initiale, avant modification, comprend également au moins une séquence d'acide aminés ci-dessous dans sa partie N-terminale (zone conservée 2) :

X10-X11-Y-X12-**R**-X13-X14-X15-X16-X17-X18

dans laquelle
X10 représente A, H,Y,F,L,K, de preference A
X11 représente Y, E,D,K,R,V,I, de preference Y
X12 représente S,A,T,P,G, de preference S
X13 représente I,S,T,R,E,F,W,D, de preference S.
X14 représente S,G,A,I,E,N,K,P, de preference G
X15 représente N,H,Q,S, de preference N
X16 représente P,D,L, de preference P
X17 représente T,M,N,G,S, de preference T et
X18 représente R,L,V,S,W,E, de preference R.

Les acides aminés X1 à X9 correspondent aux résidus 324 à 334 de la séquence de cystathionine γ-synthase de *E*, *coli* K12, représenté sur la SEQ ID NO 6.

Les acides aminés X10 à X18 correspondent aux résidus 44 à 54 de la séquence de cystathionine γ-synthase de *E. coli* K12, représenté sur la SEQ ID NO 6.

Les positions des acides aminés employées ci-dessus et ci-après sont entendues comme étant des positions relatives faites par référence à la séquence de cystathionine-γ-synthase de *E. coli* K12. L'homme du métier saura bien entendu retrouver les acides aminés correspondants sur d'autres séquences de cystathionine-γ-synthases, par l'emploi d'outils usuels d'alignement de séquences. On citera notamment le programme BLAST, qui peut être utilisé à partir du site http://www.ncbi.nlm.nih.gov/BLAST/ avec les paramètres indiqués par défaut sur ce site. L'alignement de séquence pourra être effectué tant au niveau de la protéine (SEQ ID NO 6) que de sa séquence codante, comme par exemple la séquence codante représentée sur la SEQ ID NO 5.

On peut aussi avantageusement utiliser la recherche avancée de BlastP en affinant la recherche avec un motif (PHI-BLAST). Dans ce cas on pourra prendre le motif [AGS]-[EVPT]-[STN]-L-G-[GDAHT]-[VATHN]-[ERKF]-[ST]-[LIVA]-[IVAT] pour une première zone conservée et le motif x(2)-Y-[SATPG]-R-x(2)-[NHQS]-[PDL]-[TMNGS]-[RLVSWE] pour une deuxième zone conservée où les lettres indiquées en gras correspondent aux acides aminés majoritairement présent à cette position dans la séquence, et où x correspond à n'importe quel acide aminé. Le chiffre 2 entre parenthèse signifie qu'il y a deux acides aminés indéterminés.

Pour l'alignement des séquences, on peut utiliser les programmes CLUSTALW (http://www.ebi.ac.uk/clustalw/) ou MULTALIN ((http://prodes.toulouse.inra.fr/multalin/cgi-bin/multalin.pl), avec les paramètres indiqués par défaut sur ces sites.

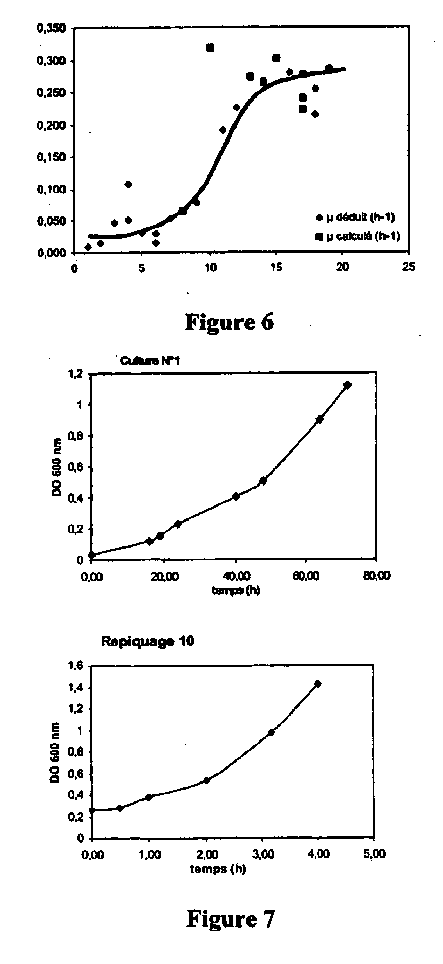

Un tel alignement de séquences est représenté sur la figure 7 pour une sélection de différentes cystathionine-γ-synthases.

Elles sont de préférence choisies parmi les cystathionine-γ-synthase (CGS) suivantes :
Q9ZMW7 O-succinylhomoserine (Thiol)-lyase, Helicobacter pylori
P46807 O-succinylhomaserine (Thiol)-lyase, Mycobacterium leprae
AA029646 Xylella fastidiosa Temeculal
NP_638204 Xanthomonas campestris pv. campestris str. ATCC 33913
NP_358970 Streptococcus pneumoniae R6
NP_126586 O-succinylhomoserine (Thiol)-lyase, Pyrococcus abyssi
NP_373671 Staphylococcus aureus subsp. aureus N315
NP _418374 [Escherichia coli K12
NP_601979 Corynebacterium glutamicum ATCC 13032
NP_343729 O-succinylhomoserine (thiol)-lyase, Sulfolobus solfataricus
NP_{_}786043 O-succinylhomoserine (thiol)-lyase, Lactobacillus plantarum WCFS1
NP_719586 Shewanella oneidensis MR-1
CAD30944 Streptomyces coelicolor A3(2)
NP_696324 Bifidobacterium longurn NCC2705
NP_457953 Salmonella enterica subsp. enterica serovar Typhi
NP_539021 Brucella melitensis
EAA30199 O-succinylhomoserine (Thiol)-lyase, Neurospora crassa
BAC61028 Vibrio parahaemolyticus,

Selon un mode préférentiel de réalisation de l'invention, la cystathionine-γ-synthase à activité « methionine synthase » modifiée est une cystathionine-γ-synthase, modifiée pour permettre la conversion directe de la O-succinyl-L-homosérine en L-methionine avec le méthyl-mercaptan comme source de soufre (composé de formule générale (II) dans lasquelle R représente le méthyle).

Selon un mode de réalisation préférentiel de l'invention, la cystathionine-γ-synthase à activité « methionine synthase » modifiée comprend la séquence d'acides aminés représentée sur la SEQ ID NO 8. Une séquence d'ADN codant pour cette enzyme modifiée est représentée sur la SEQ ID NO 7.

La présente invention concerne également les cystathionine-γ-synthases modifiées telles que définies ci-dessus ainsi que les séquences d'acide nucléique codant pour ces cystathionine-γ-synthases modifiées, notamment les séquences isolées, en particulier les séquences d'ADN et notamment la séquence représentée sur la SEQ ID NO 7, les vecteur de clonage et/ou d'expression comprenant les dites séquences, en particulier les vecteurs comprenant lesdites séquences d'acide nucléique sous le contrôle d'éléments de régulation nécessaires à l'expression et la transcription de la cystathionine-γ-synthase modifiée dans un organisme hôte et les organismes hôtes transformés avec lesdits vecteurs.

Selon un mode préférentiel de réalisation de l'invention, la modification de la voie de biosynthèse de la méthionine nécessite dans un premier temps de modifier génétiquement la bactérie initiale. La modification implique nécessairement l'atténuation d'au moins un gène et éventuellement le clonage d'au moins un gène hétérologue. L'atténuation du gène doit rendre la bactérie dépendante de la restauration d'une voie métabolique équivalente afin de permettre sa croissance. La bactérie génétiquement modifiée est cultivée et l'on sélectionne la ou les souches bactériennes dont le taux de croissance s'améliore en présence ou non d'un co-substrat exogène.

Le procédé de préparation de souches selon l'invention consiste à obtenir, à partir d'une souche bactérienne initiale, une souche bactérienne génétiquement modifiée présentant au moins une modification dans un gène codant pour une enzyme à activité « méthionine synthase » (*e.g*. MetE chez *E. coli*), ledit procédé comprenant une étape consistant à soumettre ladite souche bactérienne initiale à une pression de sélection en présence de la source de soufre définie ci-dessus, afin de diriger une évolution d'au moins un gène (*e.g. met*B chez *E. coli*) dans ladite souche bactérienne, afin de restaurer une activité «méthionine synthase» ou «homocystéine synthase» dans la souche évoluée; cette restauration d'activité n'étant pas liée à une réversion de la modification réalisée.

L'invention concerne donc également une souche à activité « méthionine synthase » améliorée qui comprend une inactivation d'au moins un gène endogène impliqué dans la voie de biosynthèse habituelle de la méthionine.

Dans un mode préféré d'application de l'invention, ladite modification de la souche initiale, que l'on retrouve dans la souche à activité « méthionine synthase améliorée », notamment *E*. *coli,* consiste à inactiver l'activité méthionine-synthase native (codée par le gène *met*E chez *E. coli*) puis à cultiver ledit microorganisme modifié obtenu sur un milieu défini dépourvu de méthionine, S-adenosylméthionine, homocystéine, et cystathionine mais en présence de methylmercaptan (co-substrat exogène), ou de l'un de ses sels n'ayant pas de propriété mutagène, et de sélectionner un microorganisme évolué caractérisé en ce que l'activité méthionine-synthase de la cystathionine γ-synthase se soit fortement améliorée, en présence de methylmercapatan, au point de remplacer l'activité initialement inactivée. Dans ce mode d'application de l'invention il est important de noter que le milieu utilisé pour sélectionner le microorganisme évolué est identique au milieu sur lequel pousse le microorganisme initial (c'est à dire non modifié) ; seul un co-substrat (*e.g.* alkylmercaptan) est ajouté. Selon un mode préférentiel de réalisation de l'invention, la souche bactérienne sélectionnée comprenant le microorganisme évolué selon l'invention, est une souche de *E. coli,* plus préférentiellement la souche *E*. *coli* K183, déposée sous le numéro 1-3005, le 2 avril 2003 à la Collection Nationale de Culture et Microorganismes (CNCM), 25 rue du Docteur Roux, 75724 "Paris Cedex 15, France, selon les dispositions du traité de , ledit procédé comprenant une étape consistant à soumettre ladite souche bactérienne initiale à une pression de sélection en présence de la source de soufre définie ci-dessus, afin de diriger une évolution d'au moins un gène (*e.g. met*B chez *E. coli*) dans ladite souche bactérienne, afin de restaurer une activité «méthionine synthase» ou «homocystéine synthase» dans la souche évoluée; cette restauration d'activité n'étant pas liée à une réversion de la modification réalisée. Budapest. Cette souche comprend un gène exprimant une cystathionine-γ-synthase modifiée, l'enzyme comprenant la mutation E325A, et une inactivation du gène *met*E*.*

Une mutation du géne *met*J a été proposée dans JP 2000157267-A/3, pour produire une quantité supérieure de méthionine (voir aussi GenBank E35587). Ce gène code pour une protéine de répression des gènes *met* B, E, L, J et R (chez *Salmonella typhimurium*). Son inactivation ou sa modification permet de diminuer le rétrocontrôle par la méthionine.

Le gène *met*C (GenBank M12858), code la cystathionine-β-lyase (EC 4.4.1.8), les gènes *met*E (GenBank AE000458) et *met*H (GenBank J04975) codent la méthionine synthase (EC 2.1.1.13). La méthionine est un acide aminé essentiel à la vie cellulaire. L'inactivation d'un ou plusieurs de ces gènes revient à supprimer la voie habituelle de biosynthèse de la méthionine.

En utilisant les références données sur GenBank pour ces gènes qui sont bien connus, l'homme du métier est capable de déterminer les gènes équivalents dans d'autres souches bactériennes qu'*E*. *coli.* Ce travail de routine est avantageusement effectué en utilisant les séquences consensus pouvant être déterminées du fait de la synthèse de ces gènes pour d'autres microorganismes, et en dessinant des sondes dégénérées permettant de cloner le gène correspondant dans un autre organisme. Ces techniques de routine de biologie moléculaire sont bien connues dans l'art et sont décrites par exemple dans Sambrook et al. (1989 Molecular cloning: a laboratory manual. 2nd Ed. Cold Spring Harbor Lab., Cold Spring Harbor, New York.).

La souche à activité « methionine synthase » modifiée selon l'invention comprenant une enzyme à activité « méthionine synthase » modifiée définie ci-dessus comprend au moins une inactivation du gène *met*E.

Lorsque l'enzyme à activité « méthionine synthase » modifiée définie ci-dessus permet d'effectuer la conversion directe du substrat de formule générale (III) en acide de formule générale (1), la souche selon l'invention comprend au mois une inactivation du gène *met*E.

Dans un mode de réalisation, ladite souche bactérienne peut comprendre également une modification de l'activité homosérine O-acyltransférase porté par le gène *metA* afin de lui conférer au choix une activité homosérine O-succinyltransférase (EC 2.3.1.46) ou homosérine O-acétyltransférase (EC 2.3.1.11).

Dans un mode particulier, on pourra remplacer ou modifier le gène *metA* de *E. coli,* codant l'enzyme possédant l'activité homosérine O-succinyltransférase (Genbank AAN83396), afin d'obtenir une activité homosérine O-acétyltransférase. Il est connu de l'homme du métier que cette activité est codée par le gène *met*A de *C*, *glutamicum* (Genbank AF052652). Les protocoles permettant de remplacer le gène *metA* de *E*. *coli* par le gène *met*A de *C. glutamicum,* ou de modifier la séquence de *met*A de *E. coli* afin d'obtenir une activité homosérine O-acetyltransferase au lieu d'une activité homosérine O-succinyltransférase sont connus de l'homme du métier.

De manière similaire on peut remplacer ou modifier le gène *met*A de *C. glutamicum,* codant une activité homosérine O-acetyltransférase, afin d'obtenir une activité homosérine O-succinyltransférase.

Toutes les modifications mentionnées ci-dessus peuvent être effectuées directement sur la souche objet de la pression de sélection, lorsque le procédé selon l'invention est mis en oeuvre. Alternativement, il est préférable de mettre en oeuvre le procédé de criblage selon l'invention sur une souche ne présentant qu'un nombre restreint de modifications, d'obtenir une souche présentant une activité « méthionine synthase » en présence du composé de formule (II), en particulier de méthyl-mercaptan, et d'effectuer alors d'autres modifications telles que mentionnées, afin d'augmenter le 'bypass' de la voie classique de synthèse de la méthionine.

L'homme du métier connaît les protocoles permettant de modifier le caractère génétique de microorganismes. La surexpression d'un gène peut être effectuée par changement du promoteur de ce gène *in situ,* par un promoteur fort ou inductible. De façon alternative, on introduit, dans la cellule, un plasmide réplicatif (simple ou multicopies) dans lequel le gène que l'on désire surexprimer est sous le contrôle du promoteur adéquat.

L'inactivation d'un gène se fait préférentiellement par recombinaison homologue. Le principe d'un protocole en est rappelé brièvement : on introduit dans la cellule un fragment linéaire, obtenu *in vitro,* comprenant les deux régions flanquant le gène, et au moins un gène de sélection entre ces deux régions (généralement un gène de résistance à un antibiotique), ledit fragment linéaire présentant donc un gène inactivé. On sélectionne les cellules ayant subi un événement de recombinaison et ayant intégré le fragment introduit par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaison.

Selon un mode préférentiel de réalisation de l'invention, la souche bactérienne est la souche *E. coli* K183, déposée à la CNCM le 2 avril 2003 sous le numéro i-3005. Cette souche comprend un gène exprimant une cystathionine-γ-synthase modifiée, l'enzyme comprenant la mutation E325A, décrite précédemment et une inactivation du gène *met*E.

Les souches de microorganismes selon l'invention possèdent une enzyme cystathionine-γ-synthase; elles sont de préférence sélectionnées et améliorées par un procédé de criblage et d'évolution, qui est aussi un objet de l'invention. Les souches selon l'invention peuvent également être génétiquement modifiées (c'est-à-dire présenter une inactivation, une mutation et/ou la suractivatian d'au moins un gène endogène), la modification étant effectuée préalablement ou non à la mise en oeuvre du procédé de criblage.

Afin d'accélérer la sélection et l'évolution dirigée des souches pour la production de méthionine en présence de composé de formule (II), en particulier de méthyl-mercaptan, on peut effectuer les opérations ci-dessous. Le procédé est décrit pour le méthyl-mercaptan.

### a. Coupler la biosynthèse de la molécule d'intérêt à la croissance du microorganisme de telle sorte que la production de cette molécule est nécessaire pour une bonne croissance du microorganisme

Pour cette raison on peut faire le choix de détruire le gène *met*E codant la méthionine-synthase qui produit la méthionine à partir d'homocystéine. Ce faisant la souche devient auxotrophe pour la méthionine.

Le microorganisme, pour vivre en milieu minimum contenant une source de carbone simple et du méthylmercaptan ou méthylmercaptide de sodium, doit donc optimiser la voie de synthèse de la L-méthionine à partir de l'O-acyl-L-homosérine et du méthylmercaptan ou méthylmercaptide de sodium. Une modélisation informatique montre que dans ces conditions il est possible de doubler les rendements théoriques en méthionine (Tableau 1).

**Tableau 1 : rendements théoriques maximums pour la production de méthionine (g de produit/g de glucose) par E. coli dans le cas d'une fermentation sur glucose (a) et d'une fermentation sur glucose et méthyl-mercaptan (b) avec un rendement en biomasse constant (cultures continues).**

| Rendements biomasse Y_{X/S} (g.g⁻¹) | Rendements méthionine^{a} Y_{P/S} (g.g⁻¹) | Rendements méthionine^{b} Y_{P/S} (g.g⁻¹) |
|---|---|---|
| 0 | 0,36 | 0,74 |
| 0,11 | 0,30 | 0,62 |
| 0,28 | 0,21 | 0,42 |
| 0,44 | 0,12 | 0,24 |
| 0,61 | 0 | 0 |

Cependant, lorsque l'on souhaite produire un acide 2-amino-4-(alkylmercapto)butyrique différent de la L-méthionine, il est nécessaire de supplémenter le milieu en méthionine pour permettre la croissance du microorganisme.

### b. Supprimer les régulations, notamment les rétro-inhibitions soit au niveau des enzymes, soit au niveau des gènes afin que la voie de biosynthèse principale soit potentialisée

On peut ainsi supprimer le gène *met*J codant une protéine répresseur. Par ailleurs, il a été montré que l'homosérine trans-succinylase, codée par le gène *met*A*,* était rétro-inhibée par la méthionine et la S-adénosylméthionine (Taylor et al., 1966, J. Biol. Chem., 241: 3641-3642). Il est donc souhaitable de remplacer cette enzyme par une enzyme insensible à la rétro-inhibition (Chater et al, 1970, J. Gen. Microbiol. 63: 121-131)

Un autre objet de l'invention est un test de criblage-identification permettant d'obtenir un microorganisme produisant de l'acide 2-amino-4-(alkylmercapto)butyrique, notamment la L-méthionine, en métabolisant un composé soufré de formule générale (II), en particulier un alkyl-mercaptan ou l'H2S, notamment le méthyl-mercaptan.

Ainsi, la présente invention permet d'identifier des souches présentant des mutations dans leur génome, lesdites mutations permettant l'assimilation du composé de formule (II) par ladite souche, et la production dudit acide aminé de formule (I). Lesdites modifications induisent donc une modification/augmentation de l'activité « méthionine synthase » de ladite souche. On peut alors accélérer la production de la souche produisant de la méthionine de façon autonome à partir d'une source de carbone simple et de composé de formule (II).

Un autre objet de l'invention est donc un procédé de criblage d'une souche bactérienne initiale, éventuellement génétiquement modifiée, possédant un gène codant pour une enzyme cystathionine-γ-synthase en vue d'obtenir une souche bactérienne génétiquement modifiée produisant un acide aminé de formule (I), en particulier de la L-méthionine, et présentant une modification dans le gène de ladite enzyme, induisant une modification de l'activité « méthionine synthase » en présence d'un composé soufré de formule (II), présentant l'étape consistant à soumettre ladite souche bactérienne à une pression de sélection en présence du composé de formule (II), afin de diriger une évolution du gène codant pour ladite enzyme cystathionine-γ-synthase dans ladite souche bactérienne, ladite évolution consistant en une mutation pour leur permettre de réaliser de manière préférentielle la conversion directe du substrat de formule générale (III) en acide aminé de formule générale (I).

Ladite souche bactérienne initiale présente éventuellement une inactivation et/ou une suractivation, notamment par insertion d'un promoteur fort constitutif, d'au moins un gène endogène.

L'invention se rapporte également à une souche bactérienne E. coli K183 déposée sous le numéro I-3005 à la CNCM présentant une modification dans le gène de l'enzyme cystathionine-γ-synthase induisant une augmentation de l'activité « méthionine synthase » de ladite enzyme en présence du composé de formule (II), en particulier de méthyl-mercaptan. Une telle souche peut également présenter au moins une autre modification génétique, (inactivation, mutation ou surexpression d'un gène endogène), telle que mentionnée ci-dessus.

L'invention se rapporte également à un procédé de préparation d'un acide 2-amino-4(alkylmercapto)butyrique de formule (I) définie ci-dessus, dans lequel on cultive une souche E. coli K183 déposée sous le numéro I-3005 à la CNCM, en présence d'un composé soufré de formule générale (II) dans un milieu approprié, ledit milieu approprié comprenant une source de carbone simple telle que définie précédemment. Ledit acide aminé de formule (I) est la L-méthionine, et ledit composé soufré de formule (II) est le méthyl-mercaptan.

Selon l'invention, les termes « culture » et « fermentation » sont employés indifféremment pour désigner la croissance de la bactérie sur un milieu de culture approprié comprenant une source de carbone simple.

La définition des conditions de culture des microorganismes selon l'invention (fermentation) est à la portée de l'homme du métier. On fermente notamment les bactéries à une température comprise entre 20°C et 55°C, de préférence entre 25°C et 40°C, plus particulièrement d'environ 37°C pour *E. coli.*

La fermentation est généralement conduite en fermenteurs avec un milieu minéral de culture de composition connu défini et adapté en fonction des bactéries utilisées, contenant au moins une source de carbone simple ainsi que du composé soufré de formule (II).

En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992 ; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96).

De manière analogue, le milieu minéral de culture pour *C. glutamicum* pourra ainsi être de composition identique ou similaire au milieu BMCG (Liebl et al., 1989, Appl. Microbiol. Biotechnol. 32: 205-210) à un milieu tel que défini par Riedel et al. (2001, J. Mol. Microbiol. Biotechnol. 3: 573-583). ,

Les milieux peuvent être supplémentés pour compenser les auxotrophies ; ils contiennent une concentration en carbone simple adaptée en fonction du mode de culture et de production, ainsi que du composé soufré de formule (II) en concentration adaptée en fonction de l'évolution de la souche et du mode de production retenu.

Après fermentation, l'acide aminé de formule (I) est récupéré selon les méthodes usuelles, et le cas échéant, purifié,

Les techniques de récupération puis de purification des acides aminés de formule (I) dans les milieux de culture sont bien connues de l'homme du métier.

La présente invention concerne également un procédé de préparation d'un acide 2-ammo-4(alkylmercapto)butyrique de formule (I) défini précédemment, dans lequel on fait réagir substrat dérivé de formule générale (III)

R"-O-(CH₂)₂-CHNH₂-COOH (III)

dans laquelle R" représente un radical acyle, de préférence choisi parmi le radical succinyle ou le radical acétyle,
avec une enzyme à activité « méthionine synthase » modifiée telle que définie précédemment, dans un milieu réactionnel approprié comprenant un composé soufré de formule générale (II) défini précédemment.

Le milieu réactionnel approprié est un milieu usuel de réaction enzymatique, bien connu de l'homme du métier, en particulier un milieu aqueux dans lequel les substrats et l'enzyme sont en solution ou en suspension. Les conditions de mise en oeuvre de la réaction sont bien connues de l'homme du métier, afin notamment d'éviter une dénaturation substantielle de l'enzyme.

Selon un mode particulier de réalisation de l'invention, l'enzyme à activité « méthionine synthase » modifiée est présente dans une bactérie inactivée ou dans un extrait cellulaire.

Selon un autre mode particulier de réalisation de l'invention, l'enzyme à activité « méthionine synthase » modifiée est une enzyme purifiée.

Les exemples ci-après permettent d'illustrer l'invention, sans toutefois chercher à en limiter la portée.

### Description des figures

Figure 1 : synthèse de la méthionine à partir de l'homosérine, chez les bactéries.
Figure 2 : Schéma de synthèse de la méthionine selon l'invention appliqué dans *E. coli ;* une stratégie équivalente est transposable chez de nombreux microorganismes dont *C. glutamicum.* Les stratégies *met*B*** ou *met*Y** nécessitent l'utilisation d'une souche initialement au moins Δ(*met*E) tandis que les stratégies *met*B** ou *met*Y* nécessitent l'utilisation d'une souche initialement au moins *Δ*(*met*C).

### Légende :

MetA : homosérine succinyltransferase ; pourra être remplacé par une isoforme insensible à la rétro-inhibition par la méthionine ou par une isoforme homoserine acetyltransferase éventuellement insensible à la rétro-inhibition par la méthionine.
MetB : cystathionine γ-synthase
MetB* : oystathionine γ-synthase évoluée en « méthionine synthase »
MetB** : cystathionine γ-synthase évoluée en homocysteine synthase
MetY* : O-acetyl-homosérine (de *C. glutamicum)* évoluée en homocysteine synthase
MetY** : O-acetyl-homosérine (de *C. glutamicum)* évoluée en « méthionine synthase »

La voie centrale représente la voie naturelle de synthèse de la méthionine chez *E. coli.* Les autres voies indiquées correspondent aux procédés selon l'invention.

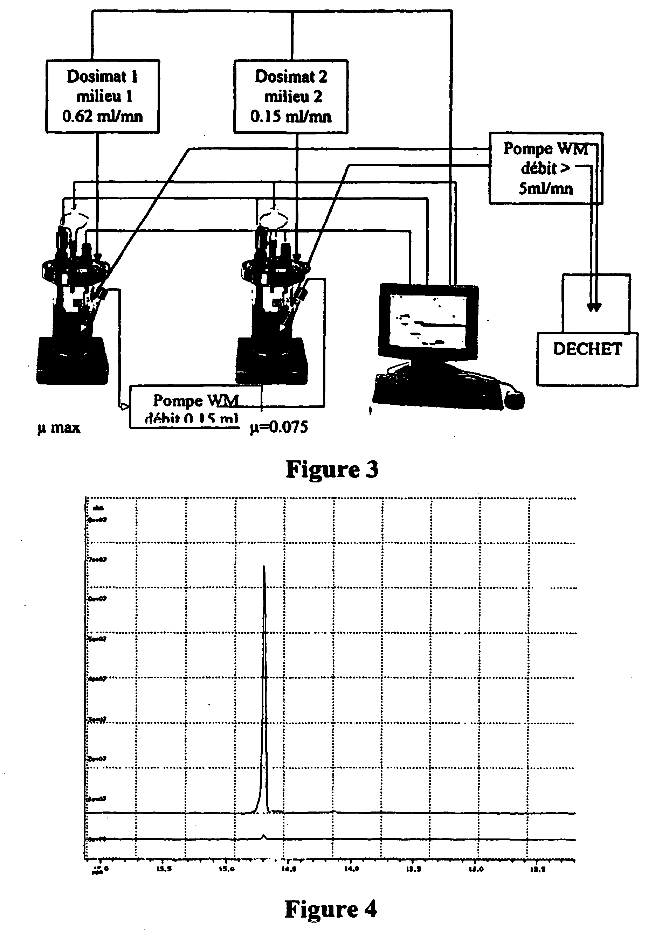

Figure 3 : représentation d'un mécanisme de fermentation continue pour la sélection dirigée des souches selon l'invention. Nous utilisons par exemple la technologie « Fedbatch-Pro » de la société DASGIP. Il s'agit d'un système modulaire contrôlé par ordinateur permettant la fermentation en parallèle de microorganismes en ayant un control de l'alimentation en milieu, en pH et pO2.

Figure 4: Comparaison de spectres de ¹³C-RMN, correspondant au carbone 5 de la méthionine, obtenus par HSQC sur un hydrolysat de la souche sauvage (haut) ou de la souche K1a-F optimisée (bas). On observe que le carbone 5 de la souche K1a-F n'est pas marqué au carbone 13 ce qui confirme qu'il provient du méthylmercaptide de sodium

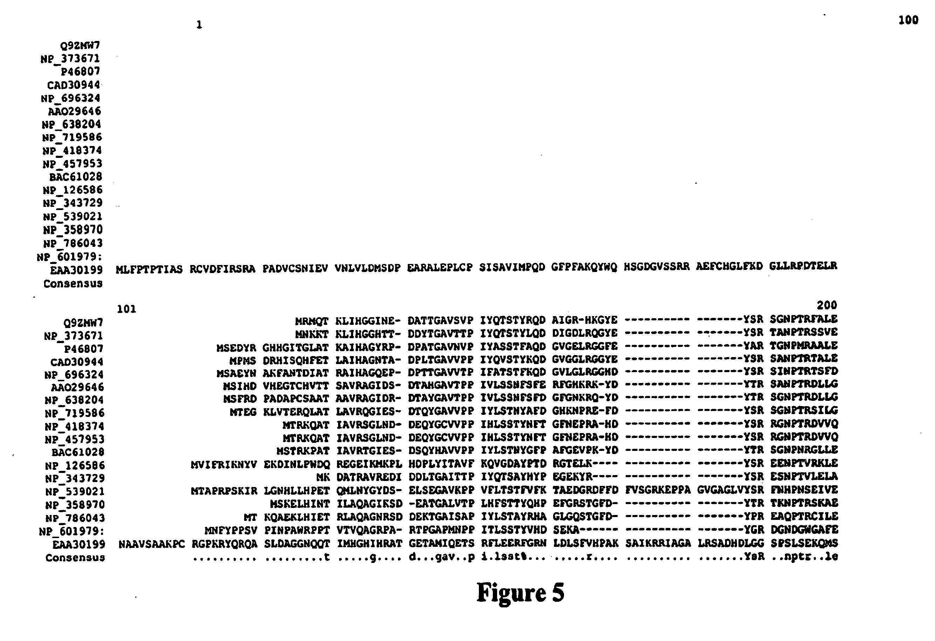

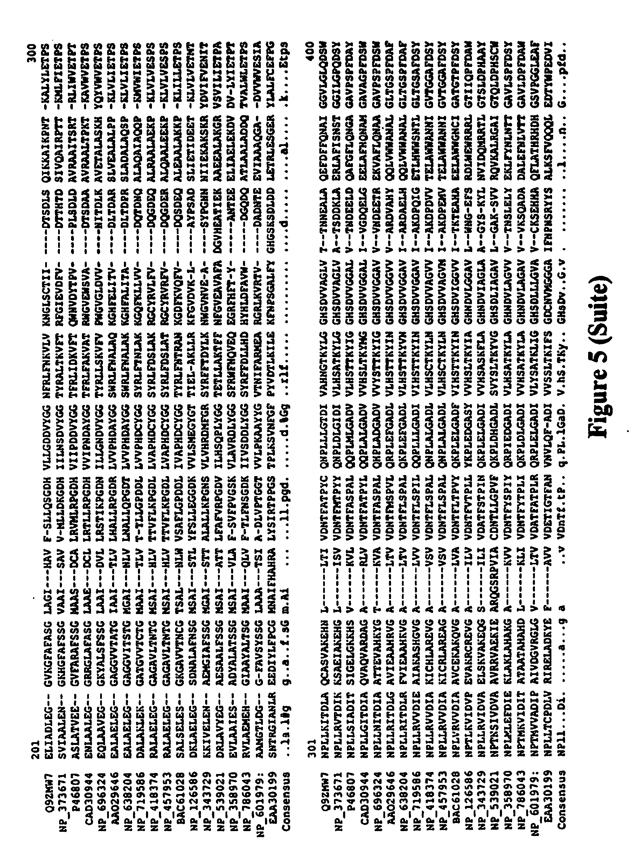

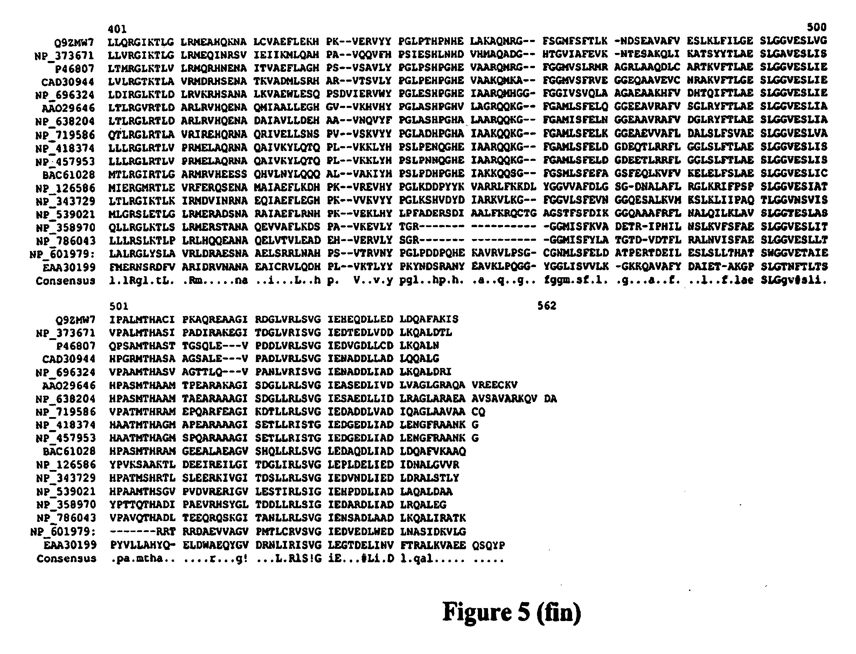

Figure 5 : Alignement de séquences non modifiées de cystathionine-γ-synthases. Alignement réalisé avec l'algorithme MULTALIN (http://prodes.toulouse.inra.fr/multalin/cgi-bin/multalin.pl)

Figure 6 : Evolution du taux de croissance de la souche initiale *E. coli Δ*(*met*E) lors du processus de sélection dirigée en batch. Abscisse : repiquage N° ; Flèche : Population K144 ; µ déduit : valeurs obtenues à partir de 2-3 valeurs de DO ; µ calculé : valeurs obtenues avec plus de 4 valeurs de DO.

Figure 7 : Cinétique de croissance de la population *E. coli* Δ*met*C lors de son ensemencement initial (culture N°1) et lors de son dixième repiquage (Repiquage 10).

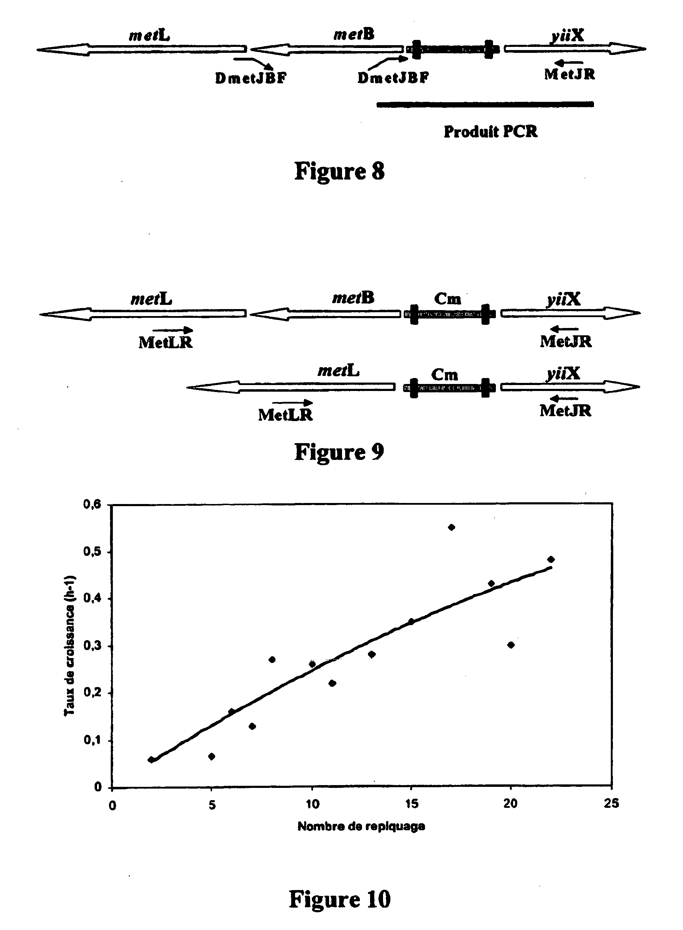

Figure 8 : Réalisation de la PCR sur la souche modifiée *E. coli* Δ*met*J ::Cm en utilisant les amorces DmetJBF et MetJR. L'extrémité 5' de l'amorce DmetJBF est aussi capable de s'hybrider à une séquence située dans la partie 3' du gène *met*L.

Figure 9 : Souches obtenues à l'issue de la recombinaison homologue avec le fragment amplifié par PCR (voir Figure 11) ; il est possible d'avoir deux évènements différents de recombinaison homologue, chacun ayant la même probabilité de se produire. Dans le premier cas, on recrée une souche *E. coli* Δ*met*J ::Cm alors que dans le second cas, on crée la souche *E. coli* [Δ*met*J*,* Δ*met*J ::Cm] en replaçant le promoteur de l'opéron metBLF devant *met*L.

Figure 10 : Evolution du taux de croissance de la souche *E. coli* [Δ(*met*BJ, *met*C) pTrc-*met*Y] sur un milieu défini (MML8), contenant du glucose pour seule source de carbone.

### Exemples

### Exemple 1 Voie de Biosynthèse de la méthionine.

Une première application (Exemple F.I.1.) de l'invention au génie métabolique de la voie de biosynthèse de la méthionine comprend les étapes suivantes :
a) Délétion du gène *met*E dans la souche initiale d*'E.coli* ; la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
b) Culture de la souche ci-avant modifiée sur le même milieu minimum (MM) auquel on ajoute le methylmercaptide de sodium (co-substrat) afin de faire évoluer une activité enzymatique endogène en activité méthionine-synthase afin de compenser l'activité enzymatique initialement délété (MetE).
c) Sélection d'une souche évoluée ayant une nouvelle activité méthionine-synthase en présence de methylmercaptide de sodium. La souche étant alors caractérisée.
d) Isolement du gène évolué codant la protéine ayant une activité enzymatique évoluée, en l'occurrence la cystathionine-γ-synthase ayant une activité méthionine-synthase améliorée.

Une deuxième application (Exemple F.I.2.) de l'invention, au génie métabolique de la voie de biosynthèse de la méthionine, comprend les étapes suivantes :
a) Délétion du gène *met*C dans la souche initiale d*'E.coli ;* la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
b) Culture de la souche ci-avant modifiée sur le même milieu minimum (MM) en absence de tout co-substrat afin de faire évoluer une activité enzymatique endogène en activité homocystéine-synthase afin de compenser l'activité enzymatique initialement délété (MetC).

Une troisième application (Exemple F.I.3.) de l'invention à l'évolution de la voie de biosynthèse de la méthionine comprend les étapes suivantes :
a) Délétion des gènes *met*C*, met*B*, met*J dans la souche initiale d*'E.coli ;* la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
a1) Introduction du gène *met*Y, un gène hétérologue issu de *C. glutamicum.* Ce gène étant destiné à évoluer d'une activité acetylhomosérine sulfhydrylase en une activité méthionine-synthase.
b) Culture de la souche modifiée *E. coli* [*metY Δ*(*met*B, *met*C, *met*J)] sur le même milieu minimum (MM) auquel on ajoute le methylmercaptide de sodium (co-substrat) afin de faire évoluer une activité enzymatique endogène en activité méthionine-synthase afin de compenser les activités enzymatiques initialement délétées (MetB, MetC).
c) Sélection d'une souche évoluée ayant une nouvelle activité méthionine-synthase en présence de methylmercaptide de sodium.

### Obtention d'une souche évoluée ayant une activité méthionine-synthase en présence de methylmercaptide de sodium.

### a) Construction de la souche modifiée E. coli Δ(metE)

L'inactivation du gène *met*E est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad. Sci. USA 97 : 6640-6645.

Deux oligonucléotides sont utilisés pour réaliser la stratégie:
- DmetER de 100 bases (SEQ ID NO 1):
   tacccccgacgcaagttctgcgccgcctgcaccatgttcgccagtgccgcgcgggtttctggccagccgcgc gttttcagCATATGAATATCCTCCTTAG
   avec :
      une région (caractères minuscules) homologue à la séquence (4012903 à 4012824) du gène *met*E (séquence 4010643 à 4012904, séquence de référence sur le site http://genolist.pasteur.fr/Colibri/)
      une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad. Sci. USA 97 : 6640-6645)
- DmetEF de 100 bases (SEQ ID NO 2):

   avec :
      une région (caractères minuscules) homologue à la séquence (4010644 à 4010723) du gène *met*E
      une région (caractères majuscules) pour l'amplification de la cassette de résistance au chloramphénicol portée par le plasmide pKD3

Les oligonucléotides DmetER et DmetEF sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides metER et metEF.
MetER (SEQ ID NO 3) : ggtttaagcagtatggtgggaagaagtcgc (homologue à la séquence de 4012978 à 4012949)
MetEF (SEQ ID NO 4) : cccggggatgaataaacttgccgccttccc (homologue à la séquence de 4010567 à 4010596)

La cassette de résistance au chloramphénicol peut ensuite être éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment.

### b) Culture et évolution de la souche modifiée Δ(metE) en présence de methylmercaptide de sodium pour co-substrat

Pour optimiser *E. coli* pour la production de méthionine à partir du méthyl-mercaptan, on effectue une sélection dirigée en flacons.

La souche *E. coli* rendue auxotrophe pour la méthionine en inactivant le gène *met*E (voir ci-avant) ne peut donc croître qu'en fabriquant sa propre méthionine, par l'utilisation du méthyl-mercaptan.

La mise en oeuvre de cette technique permet la sélection d'une souche de *Escherichia coli* dont la cystathionine-γ-synthase (EC 4.2.99.9) a développé une activité « méthionine-synthase » modifiée en présence de méthyl-mercaptan.

La sélection dirigée est conduite en flacon en verre hermétiquement fermé contenant 50 ml de milieu minéral (Schaefer et al., 1999, Anal. Biochem. 270 : 88-96) en présence de 33 mM glucose, et du chloramphénicol à une concentration finale de 25 mg/l.

Les milieux de culture sont ensemencés avec la souche *E. coli* K12 Δ*met*E à DO₆₀₀ₙₘ définie. On ensemence avec une population de bactéries suffisamment importante pour que certaines bactéries possèdent potentiellement des mutations spontanées intéressantes dans le gène *met*B, permettant d'assimiler le méthyl-mercaptan. Cette population a été obtenue par culture de la souche auxotrophe en méthionine sur milieu minimum supplémenté en méthionine.

Trois flacons reçoivent alors 100 µl d'une solution à 400 mg/l de sodium mercaptide, tandis qu'un quatrième flacon ne reçoit pas de sodium mercaptide. Les cultures sont réalisées sous agitation, à 37°C, pendant 6 jours, puis la DO₆₀₀ₙₘ est mesurée. Les résultats sont résumés dans le Tableau N°2 ci-dessous.

**Tableau 2. Mesure de la densité optique de milieux de cultures pour E. coli en présence (flacons 1-3) ou absence (flacon témoin) de sodium mercaptide.**

| | Flacon 1 | Flacon 2 | Flacon 3 | Flacon Témoin |
|---|---|---|---|---|
| D0₆₀₀ₙₘ à T=0 | 0.34 | 0.34 | 0.34 | 0.34 |
| DO₆₀₀ₙₘ à T=6 jours | 0.23 | 1.14 | 0.79 | 0.32 |

Ces résultats montrent que les flacons 2 et 3 ont permis de multiplier une souche capable d'utiliser le méthyl-mercaptan pour produire la méthionine nécessaire à sa croissance (augmentation de la densité optique).

L'activité «méthionine synthase » améliorée observée provient d'une modification dans le gène de la cystathionine γ-synthase de la souche *E. coli* K12 Δ*met*E, contenue dans les flacons 2 et 3.

La population bactérienne du Flacon 2 peut alors être utilisée pour améliorer davantage l'activité « méthionine synthase » en présence de méthyl-mercaptan, en utilisant un procédé de criblage et amélioration par fermentation en étage, ou en recommençant le procédé en flacon tel qu'il vient d'être décrit.

### c) Criblage et amélioration par fermentation en étage

Pour optimiser *E. coli* pour la production de méthionine à partir du méthyl-mercaptan, on effectue une sélection.

La mise en oeuvre de cette technique permet la sélection d'une souche de *Escherichia coli* dont la cystathionine-γ-synthase (EC 4.2.99.9) a développé une activité « méthionine-synthase » améliorée en présence de méthyl-mercaptan.

Alternativement, on peut utiliser une souche obtenue selon l'exemple 2.

La sélection dirigée est conduite dans un système en continu en étage (Figure 3).

Le premier fermenteur produit les bactéries à une vitesse proche du taux de croissance maximum. Les bactéries passent en continu de ce fermenteur dans un second fermenteur caractérisé par un taux de dilution plus faible et un milieu avec le crible de sélection (ici, le méthyl-mercaptan).

La pression de sélection, imposée à la bactérie dans le second fermenteur, est établie par la concentration en méthyl-mercaptan. Des cycles successifs de sélection permettent d'appliquer aux bactéries des cribles de plus en plus fort par des concentrations croissantes en méthyl-mercaptan.

Pour chaque concentration, la souche sélectionnée dans le second fermenteur est celle qui a évolué pour métaboliser la totalité du méthyl-mercaptan (méthyl-mercaptan résiduel nul dans le fermenteur).

Dans ce cas, on recommence la sélection en utilisant le fermenteur n°2 comme fermenteur de croissance et le fermenteur n°1 comme fermenteur de crible, présentant du méthyl-mercaptan de concentration plus forte qu'à l'étape précédente.

On effectue différents cycles de sélection pour obtenir une souche fermentant le méthyl-mercaptan avec une vitesse élevée. L'analyse de cette souche permet de définir les mutations dans le gène de la cystathionine-γ-synthase.

### d) Sélection de la souche évoluée en présence de methylmercaptide de sodium.

La population d'*E*. *coli* Δ(*met*E) issue du flacon 2 subi des repiquages successifs en flacons, permettant d'obtenir la population K1a-F. -

La nouvelle population obtenue K1a-F est mise en culture dans un milieu minimum (Schaefer et al., 1999, Anal. Biochem. 270 : 88-96) contenant 2,5 g.l⁻¹ de glucose entièrement marqué au carbone 13 et du méthylmercaptide de sodium (200 ppm) non enrichi en carbone 13. Cette population est auxotrophe pour la méthionine en l'absence de méthylmercaptide de sodium.

Après la culture, les cellules sont récupérées, lavées puis hydrolysées par HCl 6N pendant 24 heures à 107°C. Une analyse par RMN bidimensionnelle est alors réalisée (HSQC). Cette analyse permet d'étudier le carbone 5 de la méthionine, qui provient, soit de la L-cystéine, produite à partir du glucose présent dans la solution (voie classique), soit du méthylmercaptide de sodium lorsque la nouvelle voie métabolique selon l'invention est utilisée.

L'expérience est conduite de manière similaire avec la souche sauvage *E. coli* K12 (produisant la méthionine à partir du glucose), en absence de méthylmercaptide de sodium.

La figure 6 montre deux spectres 1D, issus de deux acquisitions distinctes, superposés pour une meilleure lecture. Ces spectres 1D sont extraits de spectres RMN à deux dimension type HSQC (corrélation entre protons et carbone 13). Les spectres RMN à deux dimensions ont été obtenus sur un hydrolysat acide des bactéries.

L'échantillon analysé est un hydrolysat total ; cependant du fait de la sensibilité de la RMN et des temps d'acquisition utilisés, on détecte essentiellement les acides aminés, les sucres, les bases et le glycérol. Chaque carbone (couplé à un proton) de chaque acide aminé donne une résonance magnétique nucléaire.

Le carbone 5 de la méthionine (c'est à dire le groupe méthyl terminal) présente un déplacement chimique d'environ 14,7 ppm. La figure 6 présente la zone de déplacement chimique centrée autour de 14,7 ppm pour les deux souches.

On remarque que dans le cas du spectre supérieur, le signal du carbone 5 est fort, indiquant que le carbone 5 est marqué au carbone 13. En conséquence, ce carbone 5 provient du glucose marqué introduit comme substrat dans le milieu de culture.

On remarque par contre que le même signal est très faible dans le spectre inférieur (souche K1a-F). Cela signifie que le carbone 5 n'est pratiquement pas marqué. Pourtant les autres carbones de la molécule sont fortement marqués (résultats non présentés). Le carbone 5 non marqué ne provient donc pas du glucose mais du méthyl-mercaptan.

On peut donc conclure que la souche K1a-F produit de la méthionine à partir de succinyl-L-homosérine et de méthylmercaptide de sodium.

La population K1a-F subit 14 nouveaux cycles de repiquage successifs en flacon. On obtient ainsi la population K144 (figure 9) que l'on étale alors sur milieu minimum gélosé contenant du glucose pour seule source de carbone. Les boites inoculées sont placées en condition aerobie dans une jarre anaérobie dans laquelle est introduit un tube contenant du méthylmercaptide de sodium dissout dans de l'eau, la jarre est alors placée dans un incubateur à 37°C. La température d'ébullition du methylmercaptide de sodium étant de 5°C, l'atmosphère de la jarre anaérobie s'enrichie en methylmercaptan. Après 4 jours, les clones apparaissent sur les boites ; ils correspondent aux bactéries capables de produire de la méthionine en présence de méthylmercaptan. Dix clones sont isolés, dont le clone K176. Le clone K176 est multiplié en culture liquide et des stocks glycérols sont réalisés portant le numéro K183.

Pour le clone K183 et la souche *E. coli* K12 Δ(*met*E) initiale, la séquence des gènes *met*J et *met*B (SEQ ID N°5) a été déterminée. La séquence obtenue pour *met*B évoluée, appelée *met*B* (SEQ ID N°7) permet d'observer la présence d'une alanine en position 325 (SEQ ID N°8) en remplacement d'un glutamate (SEQ ID N°6). Le gène *met*J ne présente aucune mutation. Cette souche a été déposée à la CNCM le 2 avril 2003, sous le numéro I-3005.

### • Caractérisation du clone K183

Le clone K183 est cultivé en flacon, en milieu minimum avec glucose et methylmercaptide de sodium pour seule source de carbone. En parallèle, une culture est réalisée dans des conditions identiques avec la souche *E. coli* K12 sauvage. On observe que la consommation de glucose par g de biomasse est deux fois plus importante que pour une souche de *E. coli* sauvage (MBM01). La surconsommation est probablement due en partie à la production d'acétate.

**Tableau 3. Comparaison du rendement de biomasse entre la souche E. coli sauvage et le clone évolué K183 :**

| **Souches** | **Rendement Biomasse** | **Rendement Acétate** |
|---|---|---|
| MBM01 | 0.45 | <0.002 |
| K183 | 0.24 | 0.36 |

### Rendement Biomasse exprimé en g_{(biomasse)}/g_{(glucose)}.

### Rendement Acétate exprimé en g_{(acetate)}/g_{(glucose)}.

L'analyse des métabolites intracellulaires et extracellulaire de ces deux cultures montre notamment :

En intracellulaire, une augmentation de l'alanine, du pyruvate, du ketobutyrate et de 2 ketoisocaproate et une diminution de la concentration en tryptophane, norvaline, norleucine, leucine et methionine.

En extracellulaire, une accumulation de glutamate, d'isoleucine, thréonine, valine et 2-ketoisocaproate et une diminution de pyruvate, norleucine, tryptophane.

### • Caractérisation de l'activité spécifique « méthionine synthase » des souches MBM01 et K183 en présence de méthylmercaptan.

Afin de montrer l'amélioration de l'activité méthionine synthase dans la souche K183 par rapport à la souche sauvage (MBM01), des réactions enzymatiques sont réalisées en utilisant des extraits acellulaires préparés à partir de cultures des souches K183 et MBM01 réalisées sur milieu riche (milieu BH1, commercialisé par DIFCO, avec 2,5 g/L de glucose) en absence de méthylmercaptan. Les extraits protéiques sont désalés sur PD10 et réservés sur glace.

### Conditions réactionnelles et traitement de l'échantillon

- Préparer sur la glace une solution de sodium methanethiolate diluée 10 fois (100 µl de solution 3M plus 900 µl d'eau mQ)
- Préparer sur la glace les mélanges réactionnels constitués de 20µL de tampon phosphate 500 mM pH 6.5, 10µl Pyridoxal phosphate 2,5 mM, 16 µL O-succinylhomosérine 25 mM, 10 µL Sodium methanethiolate 0,3 M, 24 µL eau milliQ.
- Placer les tubes à 37°C (thermomixer sous la hotte) et ajouter l'extrait protéique (20 µl) pour démarrer la réaction. Pour arrêter la réaction (0 à 30 minutes), placer les tubes sur glace et ajouter 400 µl d'acétone à -20°C.
- placer les tubes à -20°C pendant 30 minutes
- puis ouvrir les tubes sous la hotte pendant 10 minutes pour évaporer le méthanethiol et l'acétone (les maintenir sur glace)
- centrifuger 5 minutes à 10000 g , transvaser le surnageant (~100 µL) dans des tubes éppendorf et diluer pour un volume final de 1 mL.

### Mesure de l'activité méthionine synthase en détectant la quantité de succinate libéré du succinylhomosérine :

Dix µl de l'échantillon ci-dessus obtenu sont analysés par chromatographie ionique en utilisant un Appareil Dionex DX-500 équipé d'une précolonne AG-11 2 mm et d'une colonne AS-11 2 mm, d'un suppresseur ASRS Ultra, d'une boucle injection 10 µl. Un gradient est alors appliqué : 0 - 7 min 0,5 mM KOH ; 7 min injection ; 7 - 9,5 min 0,5 mM KOH ; 9,5 - 13 min 0,5 - 5 mM KOH ; 13 - 25 min 5 - 38,3 mM KOH.

### Mesure de l'activité méthionine synthase en détectant la quantité de méthionine synthétisée en présence de methylmercaptan

L'analyse est réalisée par GC-MS ce qui nécessite de silyler les échantillons préalablement à l'injection. Pour cela chaque échantillon reçoit un standard interne (serine13C) qui permet de valider la qualité de la silylation. Les échantillons sont ensuite lyophilisés pendant la nuit.

La dérivation est réalisée en appliquant le protocole suivant :

Ajouter 400 µl de la solution d'hydroxylamine (0,250g +/- 0,002 g dissous dans 10 ml de Pyridine) à l'aide d'une pipette automatique de 1 ml et fermer correctement les tubes. Mélanger à l'aide d'un Vortex pendant 2 fois 10 secondes. Concentrer le milieu réactionnel au fond du tube par centrifugation (maxi 1 minute à 5000 g) et laisser réagir 1 heure 30 à 30°C. Ouvrir les tubes et ajouter 1000 µl de solution de BSTFA à l'aide d'une pipette automatique de 1 ml et compléter avec 100µl de Pyridine (pipette automatique de 200 µl). Refermer les tubes, vortexer pendant 10 secondes et mettre à incuber respectivement pendant 60 minutes à 60°C dans le cas des dérivés TBDMS et 30 minutes à 70°C pour le BSTFA. Si nécessaire filtrer les échantillons sur filtre à usage unique avec membrane PTFE de 0,22 µm ou centrifuger à 5000 g pendant 5 minutes. Transférer dans un flacon de 1,5 ml, sertir et injecter en CPG.

Les analyses ont été réalisées avec l'appareil Agilent technologies - GC6890/MSD5973 équipé d'une colonne apolaire (HP-5MS, Bios Analytique). Le gaz vecteur est l'hélium à débit constant de 1 ml.min-1. L'injection de 1 µl d'échantillon a lieu en mode splitless avec un débit de purge de 50 ml.min-1 pendant 0,85 min. Le profil de température est le suivant : la température initiale de 90°C est maintenue pendant 2 minutes puis augmente jusqu'à 320 °C avec une pente de 10 °C.min-1. Cette température est maintenue pendant 6 minutes. La détection se fait par spectrométrie de masse avec ionisation par impact électronique en mode balayage dans une gamme variant de m/z = 40 à 550 amu. Le délai de passage de solvant est réglé à 3,10 minutes.

Dans ces conditions, une activité « méthionine synthase » a pu être dosée dans les échantillons incubés avec le méthanethiol, via la quantification d'une part, du succinate par chromatographie ionique et d'autre part, de la méthionine par GC-MS. Les résultats sont reportés dans le Tableau 4 ci-dessous.

**Tableau 4 : Activité méthionine synthase en présence de méthylmercaptan d'extraits des souches MBM01 et K183**

| **Souche** | **Concentration en protéines** | **Activité spécifique (mUI/mg protéines)** | |
|---|---|---|---|
| | | **Dosage Succinate** | **Dosage Méthionine** |
| **MBM01** | 3,43 | 0,30 | 0,23 |
| **K183** | 3,62 | 1,40 | 1,72 |

On observe donc que l'activité méthionine synthase en présence de methylmercaptan est renforcée dans la souche évoluée par rapport à la souche sauvage confirmant que la cystathionine γ-synthase mutée (E325A) a une activité méthionine synthase modifiée.

### e) isolement du gène évolué et caractérisation cinétique de l'enzyme METB* ayant une activité méthionine-synthase évoluée

Afin de déterminer les paramètres cinétiques des activités méthionine-synthase et cystathionine-γ-synthase portées par METB et METB*, les gènes metB et metB* ont été clonés dans un vecteur de surexpression pTopo (Invitrogene) en utilisant la stratégie suivante :
- Amplification du gène *met*B ou *met*B* avec les oligonucléotides metJ / metLR et la polymérase thermorésistante *Pwo.*
- Ligation du produit PCR au plasmide pTOPO 4-PCR blunt, et introduction du plasmide ainsi formé, pTopo.metB ou pTopo.metB* dans DH5α et sélection des clones Ap^{r}.
- Vérification par digestion enzymatique de la configuration du plasmide pTopo.metB ou pTopo.metB* après extraction.
- Introduction du plasmide vérifié pTopo.metB dans la souche MG1655(ΔmetBJ, ΔmetE). Vérification par digestion enzymatique, comme précédemment du plasmide introduit. Vérification par PCR de la souche MG1655(Δ*metBJ,* Δ*met*E) avec les oligonucléotides metJR / metLR pour la délétion metJ, metER / metEF pour la délétion metE : souche MINS33.

Les cultures sont alors réalisées sur milieu riche et des extraits protéiques sont réalisés. Les activités enzymatiques méthionine-synthase et cystathionine-γ-synthase sont ensuite déterminées en utilisant respectivement le methylmercaptide de sodium et la cystéine comme co-substrat réactionnel.
Les caractéristiques cinétiques de l'activité méthionine synthase sont présentées dans le tableau N°5 tandis que les caractéristiques cinétiques de l'activité cystathionine-γ-synthase sont présentées dans le tableau N°6.

**Tableau 5. Caractéristiques cinétiques apparentes de l'activité méthionine-synthase portée par les enzymes METB et METB*.**

| | Km | Vmax |
|---|---|---|
| pTOPOmetB | 277 mM | 13,9 mUl/mg protéines |
| PTOPOmetB* | 6 mM | 5,6 mUl/mg protéines |

On observe que la mutation A325E permet de diminuer de plus de 45 fois le Km de l'enzyme pour le methylmercaptan alors que le Vmax n'est lui diminué que d'un facteur 2.

**Tableau 6. Caractéristiques cinétiques apparentes de l'activité cystathionine-γ-synthase portée par les enzymes METB et METB*.**

| | Km | Vmax |
|---|---|---|
| pTOPOmetB | 7,5 mM | 39840 mUl/mg protéines |
| PTOPOmetB* | 0,6 mM | 2889 mUl/mg protéines |

On observe que la mutation A325E permet de diminuer d'environ 13 fois l'activité cystathionine-γ-synthase et le Kin de l'enzyme pour la cystéine.

### Exemple 2: Procédé de culture Fed-Batch pour la production et la purification de méthionine.

### Préculture :

La préculture est réalisée pendant une nuit en fiole de 500 ml contenant 50 ml de milieu minimum type M9 modifié, complété avec 2.5 g/l de glucose. Les cellules sont récupérées par centrifugation et reprises dans 5 ml de milieu minimum type M9 modifié.

### Culture en fermenteur.

La culture est réalisée dans un fermenteur de volume utile de 300ml de type Fedbatch-pro DASGIP.

Le fermenteur est rempli avec 145 ml de milieu minimum type M9 modifié et inoculer avec les 5 ml de préculture. Soit une DO600nm d'inoculation comprise entre 0.5 et 1.2.

La température de la culture est maintenue entre 30 et 37°c et le pH est ajusté en permanence à une valeur comprise entre 6.5 et 8. Il est partiellement régulé par l'ajout d'une solution CH3SNa. Une solution de soude 2N peut le cas échéant compléter la régulation. L'agitation est maintenue à entre 200 et 400 rpm pendant la phase de batch et est augmentée jusqu'à 1000 rpm en fin de fed-batch. La teneur en 02 dissout est maintenue entre 30% et 40% de saturation en utilisant un contrôleur de gaz. Dés que la OD 600nm à une valeur comprise entre 2.5 et 3 nous commençons le fed-batch par ajout du milieu de fed à un débit initial compris entre 0.3 et 0.5 ml/h avec une augmentation progressive jusqu'à des débits compris entre 2.5 et 3.5 ml/h. après ce stade le débit est maintenu constant pendant un temps compris entre 24h et 32h. Le milieu de fed est constitué sur la base d'un milieu M9 modifié complémenté par une concentration en glucose comprise entre 300 et 500g/l de glucose. Dans le même temps nous complémentons le milieu avec une solution de CH3SNa (solution entre 1 et 5M) afin de permettre la croissance de la bactérie tout en régulant le pH. Dés que la concentration cellulaire atteint une concentration comprise entre 20 et 50 g/l nous remplaçons le milieu de fed par un milieu minimum type M9 limité en phosphore. La solution de méthyl-mercaptan est remplacée par une injection directe de CH3SH sous forme gazeux dans le fermenteur. Le débit du gaz est adapté au débit de la solution de fed dans des rapports molaires avec le substrat carboné de 1 à 3. Dés que la concentration cellulaire est comprise entre 50 et 80 g/l la fermentation est arrêtée. Le mout de fermentation est ajusté à un pH compris entre 7.5 et 9 par une solution de NaOH puis chauffé à entre 60 et 80°c. Le mout est ensuite filtré sur des modules UF. La température du jus est maintenue entre 60 et 80°C, puis le jus est concentré avant passage sur charbon pour décoloration (en colonne ou en batch). Le jus décoloré est de nouveau filtré pour retirer les dernières particules avant d'être acidifié par HCl concentré jusqu'à un pH inférieur à 2.28 (pK1 de la méthionine). Les cristaux methionine.HCl ainsi formés sont récupérés par filtration, puis l'HCl est éliminé par évaporation pour purifier la L-Méthionine.

### Dépôt de matériel biologique

La Souche K183 a été déposé le 02 Avril 2003 à la Collection Nationale de Cultures de Microorganismes (CNCM), 25 rue du Docteur Roux, 75724 Paris Cedex 15, France, selon les dispositions du Traité de Budapest, sous le Numéro d'ordre 1-3005.

### REFERENCES

Anderson, E.H. (1946), Growth requirements of virus-resistant mutants of Escherichia coli strain "B" Proc. Natl. Acad. Sci. USA 32;120-128
A Baudin, O Ozier-Kalogeropoulos, A Denouel, F Lacroute, and C Cullin (1993) A simple and efficient method for direct gene deletion in Saccharomyces cerevisiae, Nucl. Acids Res., 21: 3329-3330, 1993;
Brachmann CB, Davies A, Cost GJ, Caputo E, Li J, Hieter P, Boeke JD. (1998) Designer deletion strains derived from Saccharomyces cerevisiae S288C: a useful set of strains and plasmids for PCR-mediated gene disruption and other applications. Yeast. 14:115-32.
Datsenko, K.A. ; Wanner, B.L. (2000) One-step inactivation of chromosomal genes in Escherichia coli K-12 using PCR products. Proc. Natl. Acad. Sci. USA 97 : 6640-6645
Miller, 1992 ; A Short Course in Bacterial Genetics: A Laboratory Manual and Handbook for Escherichia coli and Related Bacteria, Cold Spring Harbor Laboratory Press, Cold Spring Harbor, New York
Sambrook et al. (1989) Molecular cloning : a laboratory manual. 2nd Ed. Cold Spring Harbor Lab., Cold Spring Harbor, New York
Schaefer, U.; Boos, W.; Takors, R.; Weuster-Botz, D. (1999) Automated sampling device for monitoring intracellular metabolite dynamics., Anal. Biochem. 270 : 88-96 Wach,A., Brachat,A., Pohlmann,R., and Philippsen, P. (1994) New heterologous modules for classical or PCR-based gene disruptions in Saccharomyces cerevisiae. Yeast, 10, 1793-1808.

## Revendications

1. Procédé de préparation de microorganismes évolués d'*Escherichia coli,* permettant une modification de la voie de biosynthèse de la méthionine, **caractérisé en ce qu'**il consiste en les étapes suivantes :
a) Obtention d'un microorganisme modifié par inactivation de l'activité methionine synthase native dans les cellules d'un microorganisme initial de manière à inhiber la production de méthionine lorsque le microorganisme est cultivé sur un milieu défini, affectant ainsi la capacité de croissance du microorganisme, la modification comprenant la délétion du gène metE,
b) Culture du microorganisme modifié précédemment obtenu sur ledit milieu défini pour le faire évoluer, en présence de methylmercaptan ou de l'un de ses sels **n'ayant pas de propriété mutagène,** ledit milieu défini étant dépourvu de méthionine, S-adenosylmethionine, homocysteine et cystathionine, et
c) Sélection des cellules de microorganismes modifiés capables de se développer sur le milieu défini, en présence de methylmercaptan, les microorganismes évolués étant des microorganismes modifiés comprenant une cystathionine γ-synthase endogène à activité methionine synthase améliorée.

2. Souche E. coli K183 déposée sous le numéro I-3005 à la CNCM, capable de se développer sur ledit milieux défini en présence de methylmercaptan ou de l'un de ses sels n'ayant pas de propriété mutagène, comprenant une cystathionine γ-synthase endogène à activité methionine synthase améliorée.

3. Procédé de préparation d'une protéine évoluée consistant en une cystathionine γ-synthase activité methionino synthase améliorée, **caractérisé en ce qu'**on cultive le microorganisme évolué selon la revendication 2 dans un milieu de culture approprié pour la production de la protéine évoluée.

4. Protéine évoluée consistant en une cystathionine γ-synthase à activité methionine synthase améliorée de *E. coli,* **caractérisée en ce qu'**elle comprend une mutation E325A.

5. Cystathionine γ-synthase à activité methionine synthase améliorée selon la revendication 4, **caractérisée en ce qu'**elle compred la sequence peptidique identifiée par la SEQ ID NO 8.

6. Sequence d'acide nucléique codant pour une cystathionine γ-synthase à activité methionine synthase améliorée selon l'une des revendications 4 ou 5.

7. Sequence d'acide nucléique selon la revendication 6, **caractérisé en ce qu'**elle comprend la séquence d'acide nucléique identifiée par la SEQ ID NO 7.

8. Procédé d'obtention d'un gène évolué codant pour une protéine cystathionine γ-synthase évoluée ayant une activité methionine synthase améliorée, **caractérisé en ce qu'**il comprend la préparation d'un microorganisme *d'Escharichia coli* évolué selon revendications 2, ledit microorganisme évolué comprenant au moins un gène évolué, codant pour une protéine cystathionine γ-synthase évoluée ayant une activité methionine synthase améliorée, et **en ce qu'**il comprend une étape d) d'isolation du gène évolué codant pour ladite protéine cystathionine γ-synthase évoluée.

9. Procédé de préparation de méthionine comprenant les étapes suivantes :
a) mise en culture d'un microorganisme évolué selon la revendication 2, en présence de methylmercaptan et d'une source de carbone simple ;
b) extraction de la méthionine et le cas échéant purification.

## Patentansprüche

1. Verfahren zur Herstellung von veränderten Mikroorganismen aus *Escherichia coli,* welches eine Modifikation des Biosytheseweges von Methionin ermöglicht, **dadurch gekennzeichnet, dass** das Verfahren die folgenden Schritte umfasst:
a) Erhalten eines modifizierten Mikroorganismus durch Inaktivierung der nativen Methioninsynthase-Aktivität in den Zellen eines Ausgangsmikroorganismus, indem die Produktion von Methionin inhibiert wird, wenn der Mikroorganismus in einem definierten Medium kultiviert wird, sodass die Wachstumsfähigkeit des Mikroorganismus beeinflusst wird, wobei die Modifikation die Deletion des Gens metB umfasst,
b) Kultivieren des zuvor erhaltenen modifizierten Mikroorganismus in dem definierten Medium, um ihn sich verändern zu lassen, in Gegenwart von Methylmercaptan oder einem seiner Salze ohne mutagene Eigenschaften, wobei das definierte Medium kein Methionin, S-Adenosylmethionin, Homocystein und Cystathion enthält, und
c) Selektieren von Zellen von modifizierten Mikroorganismen, die in der Lage sind, sich in dem definierten Medium in Gegenwart von Methylmercaptan zu entwickeln, wobei die veränderten Mikröorganismen modifizierte Mikroorganismen sind, welche eine endogene Cystathion-γ-Synthase mit verbesserter Methioninsynthase-Aktivität umfassen.

2. Stamm *E. coli* K183, hinterlegt unter der Nummer I-3005 bei der CNCM (Collection Nationale de Cultures de Microorganismes), welcher in der Lage ist, sich in dem definierten Medium in Gegenwart von Methylmercaptan oder einem seiner Salze ohne mutagene Eigenschaften zu entwickeln, umfassend eine endogene Cystathion-γ-Synthase mit verbesserter Methioninsynthase-Aktivität.

3. Verfahren zur Herstellung eines veränderten Proteins, welches aus einer Cystathion-γ-Synthase mit verbesserter Methioninsynthase-Aktivität besteht, **dadurch gekennzeichnet, dass** veränderte Mikroorganismen nach Anspruch 2 in einem geeigneten Kulturmedium kultiviert werden, um das veränderte Protein herzustellen.

4. Verändertes Protein, welches aus einer Cystathion-γ-Synthase mit verbesserter Methioninsynthase-Aktivität aus *E. coli* besteht, **dadurch gekennzeichnet, dass** es eine Mutation E325A umfasst.

5. Cystathation-γ-Synthase mit verbesserter Methioninsynthase-Aktivität nach Anspruch 4, **dadurch gekennzeichnet, dass** sie die durch SEQ ID NO 8 identifizierte Peptidsequenz umfasst.

6. Nukleinsäuresequenz, welche für eine Cystathion-γ-Synthase mit verbesserter Methioninsynthase-Aktivität nach einem der Ansprüche 4 oder 5 kodiert.

7. Nukleinsäuresequenz nach Anspruch 6, **dadurch gekennzeichnet, dass** sie die durch SEQ ID NO 7 identifizierte Nukleinsäuresequenz umfasst.

8. Verfahren zum Erhalten eines veränderten Gens, welches für ein verändertes Cystathion-γ-Synthase-Protein mit einer verbesserten Methioninsynthase-Aktivität kodiert, dadurch gekennezeichnet, dass das Verfahren die Herstellung eines veränderten Mikroorganismus aus *Escherichia coli* nach Anspruch 2 umfasst, wobei der veränderte Mikroorganismus mindestens ein verändertes Gen umfasst, welches für ein verändertes Cystathion-γ-Synthase-Protein mit einer verbesserten Methioninsynthase-Aktivität kodiert, und dass es einen Schritt d) des Isolierens des veränderten Gens, welches für das veränderte Cystathion-γ-Synthase-Protein kodiert, umfasst.

9. Verfahren zur Herstellung von Methionin, umfassend die folgenden Schritte:
a) Kultivieren eines veränderten Mikroorganismus nach Anspruch 2 in Gegenwart von Methylmercaptan und einer einfachen Kohlenstoffquelle;
b) Extrahieren und gegebenenfalls Reinigen des Methionins.

## Claims

1. Method for the preparation of evolved *Escherichia* coli micro-organisms permitting a modification of the methionine biosynthesis pathway, **characterised in that** it consists of the following steps:
a) The preparation of a modified micro-organism by inactivating the native methionine synthase activity in the cells of an initial micro-organism so as to inhibit the production of methionine when the micro-organism is grown on a defined medium, thereby impairing the ability of the micro-organism to grow, the modification comprising metE gene deletion,
b) The culture of the modified micro-organism thereby obtained on said defined medium to cause same to evolve, in the presence of methyl mercaptan or any of the salts thereof not having mutagenic properties, said defined medium being devoid of methionine, S-adenosyl methionine, homocysteine and cystathionine, and
c) The selection of modified micro-organism cells capable of growing on said defined medium, in the presence of methyl mercaptan, the evolved micro-organisms being modified micro-organisms comprising an endogenous cystathionine γ-synthase with enhanced methionine synthase activity.

2. The strain *E. coli* K183 registered under the number I-3005 at the CNCM, capable of growing on said defined medium in the presence of methyl mercaptan or any of the salts thereof not having mutagenic properties, comprising an endogenous cystathionine γ-synthase with enhanced methionine synthase activity.

3. Method for the preparation of an evolved protein, consisting of a cystathionine γ-synthase with enhanced methionine synthase activity, **characterised in that** the evolved micro-organism according to claim 2 is grown in a culture medium appropriate for the production of the evolved protein.

4. Evolved protein consisting of *E*. *coli* cystathionine γ-synthase with enhanced methionine synthase activity, **characterised in that** it comprises an E325A mutation.

5. Cystathionine γ-synthase with enhanced methionine synthase activity according to claim 4, **characterised in that** it comprises the peptide sequence identified by SEQ ID No 8.

6. Nucleic acid sequence coding for a cystathionine γ-synthase with enhanced methionine synthase activity according to any of claims 4 or 5.

7. Nucleic acid sequence according to claim 6, **characterised in that** it comprises the nucleic acid sequence identified by SEQ ID NO 7.

8. Method for the preparation of an evolved gene coding for an evolved cystathionine γ-synthase protein with enhanced methionine synthase activity, **characterised in that** it comprises the preparation of an evolved *Escherichia coli* micro-organism according to claim 2, said evolved micro-organism comprising at least one evolved gene, coding for a cystathionine γ-synthase protein with enhanced methionine synthase activity, and **in that** it comprises a step d) for isolating the evolved gene coding for said evolved cystathionine γ-synthase protein.

9. Method for the preparation of methionine comprising the following steps:
a) the placing in culture of an evolved micro-organism according to claim 2, in the presence of methyl mercaptan and a simple carbon source;
b) the extraction of methionine and purification, if required.
